# EUROPÄISCHE PATENTANMELDUNG

(11) **EP 3 834 829 A2**
(43) Veröffentlichungstag der Anmeldung: **16.06.2021**
(21) Anmeldenummer: 21150095.4
(22) Anmeldetag: 02.12.2016
(51) Int. Cl.: A61K 31/505, A61P 15/00, A61P 17/00, A61P 39/00, A61P 43/00

(54) **KOMPATIBLES SOLUT ODER SOLUTGEMISCH (VORZUGSWEISE ECTOIN BZW. ECTOIN-DERIVATE) ZUR VERWENDUNG BEI DER PRÄVENTION ODER BEHANDLUNG VON KRANKHEITEN MIT BARRIEREDEFEKTEN IN EPITHELGEWEBEN**

(30) Priorität: 03.12.2015 DE 102015121050
(62) Teilanmeldung aus: 16805420.3
(71) Anmelder: Bitop AG, Witten 58453 (DE)
(72) Erfinder: BILSTEIN, Andreas, Dr., 50129 Bergheim (DE); SCHERNER, Olaf, Dr., 33729 Bielefeld (DE)
(74) Vertreter: Heide, Anna Katharina

(57) **Zusammenfassung**

Die vorliegende Erfindung betrifft ein kompatibles Solut oder Solutgemisch sowie eine Zusammensetzung enthaltend mindestens ein Solut oder ein Solutgemisch (vorzugsweise Ectoin bzw. Ectoin-Derivat) zur Verwendung bei der Prävention oder Behandlung von mit mindestens einer biobasierten Noxe assoziierter Barrieredefekte von Übergangsepithelgewebe umfassend Genitalien, enthaltend mindestens eine Verbindung ausgewählt aus Verbindungen der Formel I, der Formel II, den physiologisch verträglichen Salzen der Formel I und Formel II, den stereoisomeren Formen der Verbindungen der Formel I, Formel II und der physiologisch verträglichen Salze der stereoisomeren Formen oder einem Gemisch aus mindestens zwei der vorgenannten Verbindungen. Das mindestens eine kompatible Solut, Solutgemisch und/oder die Zusammensetzung werden in Form eines Kosmetikums, Medizinproduktes, Arzneimittels, eines Zusatzes zu einem der vorgenannten Erzeugnisse oder als Bestandteil eines in-vitro-Diagnostikprodukts (IVD) bereitgestellt.

## Beschreibung

Die vorliegende Erfindung betrifft ein kompatibles Solut oder Solutgemisch sowie eine Zusammensetzung enthaltend mindestens ein Solut oder ein Solutgemisch zur Verwendung bei der Prävention oder Behandlung von mit mindestens einer biobasierten Noxe assoziierter Barrieredefekte von Epithelgeweben, insbesondere von Krankheiten umfassend mindestens einen Barrieredefekt in mindestens einer Zelllage von mindestens einem Epithelgewebe, wobei mindestens ein Solut ausgewählt wird aus Verbindungen der Formel I, der Formel II, den physiologisch verträglichen Salzen der Formel I und Formel II, den stereoisomeren Formen der Verbindungen der Formel I, Formel II und der physiologisch verträglichen Salze der stereoisomeren Formen oder einem Gemisch aus mindestens zwei der vorgenannten Verbindungen. Das mindestens eine kompatible Solut, Solutgemisch und/oder die Zusammensetzung werden in Form eines Kosmetikums, Medizinproduktes, Arzneimittels, eines Zusatzes zu einem der vorgenannten Erzeugnisse oder als Bestandteil eines in-vitro-Diagnostikprodukts (IVD) bereitgestellt.

Kompatible Solute, auch als Osmolyte bezeichnet, sind organische Verbindungen mit geringer molarer Masse. Sie werden als Schutzstoffe in kosmetischen Zusammensetzungen sowie in Medizinprodukten und Arzneimitteln eingesetzt.

Der Mensch ist ständigen Einflüssen aus der Umwelt ausgesetzt. Einflüsse aus der Umwelt wirken als belebte und unbelebte Faktoren auf den Menschen ein. Belebte Faktoren sind z.B. Insekten, Parasiten und Schädlinge, Haustiere und Pflanzen, die durch Einwirkungen auf den menschlichen Organismus zu einer gesundheitlichen Beeinträchtigung des Menschen führen können. Unbelebte Faktoren sind Bestandteile oder Verbindungen aus der Umwelt bzw. aus der Natur. Häufig sind unbelebte Faktoren z.B. Absonderungen der belebten Faktoren, wie Kot, Toxine oder Bestandteile der belebten Faktoren, wie Pollen, Nadeln, Sekrete etc. Diese können ebenfalls, wenn Sie in Kontakt mit dem menschlichen Organismus kommen, zu einer gesundheitlichen Beeinträchtigung des Menschen führen. Insgesamt können die vorgenannten belebten und unbelebten Faktoren als biobasierte Noxen zusammengefasst werden.

Die Belastung des menschlichen und tierischen Organismus durch biobasierte Noxen ist allgegenwärtig. Auf dem Weg zur Arbeit, am Arbeitsplatz, in der Freizeit und im Urlaub ist die Umwelt, der Raum, die Luft und das Wasser mit einer Vielzahl unterschiedlicher biobasierter Noxen angereichert. Die Verwendung von Lüftungssystemen, welche die Außenluft ins Innere leiten oder durch Klimaanlagen, wird die Zirkulation und Verteilung von biobasierten Noxen gefördert. Auch durch saisonale Unterschiede variiert die Belastung mit biobasierten Noxen abhängig von der Jahreszeit, Temperatur, Wind und/oder Feuchtigkeit.

Durch biobasierte Noxen können unterschiedliche Krankheiten mit typischen Symptomen ausgelöst werden. Häufig zeigen sich Reaktionen auf, unter und/oder in der Haut. Eine Reaktion der Haut oder allgemein Hautveränderung wird auch als Effloreszenz bezeichnet. Effloreszenzen sind primär sichtbare und/oder tastbare Grundelemente einer Erkrankung der Haut und/oder der Subkutis und umfassen primäre Effloreszenzen (oder Primäreffloreszenzen), d.h. Effloreszenzen, die ohne Zwischenstadium aus der gesunden Haut entstanden sind und sekundäre Effloreszenzen (oder Sekundäreffloreszenzen), also Effloreszenzen, die aus Primäreffloreszenzen hervorgegangen sind.

Wurden die biobasierten Noxen aufgenommen, insbesondere über die Haut, Schleimhaut und/oder systemisch, zeigen sich die daraus folgenden Krankheiten an Symptomen wie z.B. Übelkeit, Durchfall, Fieber, Ödeme, Rötungen usw. Die Krankheiten umfassen oberflächliche Infektionen, innere Entzündungen, Lungenentzündungen, Asthma, Krankheiten der Bronchien, Krankheiten der Unterhaut, Krankheiten der Magen-Darm-Schleimhaut usw.

Gemeinsam haben die durch biobasierte Noxen ausgelösten Krankheiten, dass das jeweilige Epithelgewebe zumindest teilweise geschädigt ist. Die häufigste Ursache ist eine gestörte, defekte oder zerstörte Barrierefunktion (selektive Permeabilitätsbarriere) des betroffenen Epithelgewebes. Epithel ist das Gewebe, das die inneren und äußeren Oberflächen des Körpers auskleidet und die Funktion einer Barriere für Fremdstoffe, wie z.B. biobasierte Noxen, erfüllt. Daher fungieren Epithelzellen bzw. Epithelgewebe als Barriere gegen mechanische Verletzungen, eindringende Noxen, Flüssigkeitsverlust und Verdunstung.

Charakteristisch für einzelne Epithelzellen und Epithelgewebe ist ihre Polarität. Das Epithel (synonym Epithelgewebe) besteht aus polaren Zellen, die eine basale (synonym basolaterale) Seite und eine apikale (Lumen) Seite aufweisen. Auf der basalen Seite stehen sie mit der Basalmembran und seitlich über Zellkontakte mit anderen Zellen in Verbindung. Epithelgewebe weisen keine Blutgefäße auf, aber der Nachweis von Zytokeratinen ist charakteristisch für die unterschiedlichen Epithelgewebe.

Die durch die Epithelzellen erzielte Barriere, insbesondere selektive Permeabilitätsbarriere, und der Zusammenhalt werden durch die interzellularen Verbindungen gestärkt. Man unterscheidet vier Gruppen von Zellverbindungen, die an der Bildung der Barriere im Epithelgewebe, insbesondere selektive Permeabilitätsbarriere, beteiligt sind: Tight junctions (Zonula occludens), Adhaerens junctions oder Gürteldesmosomen (Zonula adhaerens), Desmosomen und Gap junction. Der sogenannte epitheliale Schlussleistenkomplex wird durch eine Einheit (Komplex) aus Zonula occludens, Zonula adhaerens und Desmosom gebildet und kommt in den meisten einschichtigen Epithelien vor. Durch den epithelialen Schlussleistenkomplex wird in dem Raum zwischen den Körperzellen (synonym Interzellularraum) eine selektive Permeabilitätsschranke gewährleistet, die einen unkontrollierten parazellulären Stofftransport zwischen den Körperzellen verhindert. Dadurch wird das Eindringen von biobasierten Noxen kontrolliert bzw. verhindert.

Weist die vorbeschriebene Barriere, insbesondere selektive Permeabilitätsbarriere, Lücken auf, oder sind die eindringenden Noxen so klein, dass diese dennoch die Barriere passieren können, sind im Sinne der Erfindung gesundheitliche Beeinträchtigungen die Folge.

Zur Behandlung solcher gesundheitlichen Beeinträchtigung stehen dem betroffenen Menschen unterschiedliche Arzneimittel, Medizinprodukte und Kosmetika zur Verfügung. Häufig enthalten diese Präparate Steroide, Antibiotika, Antimykotika, Analgetika und/oder weitere synthetische Wirkstoffe und Hilfsstoffe. Die vorgenannten Präparate behandeln die Infektion z.B. durch Angriffe auf die Parasiten, Bakterien, Viren oder Pilze. Die Wiederherstellung der Barrieren der betroffenen Epithelgewebe wird durch diese Wirkstoffe jedoch nicht gefördert. Stattdessen müssen körpereigene Mechanismen, das Immunsystem und spezifische Expressionsfaktoren, die Barriere wiederherstellen. Ferner haben die vorgenannten Stoffklassen den Nachteil, dass diese häufig starke Nebenwirkungen aufweisen und/oder allergische Reaktionen beim Menschen auslösen können.

Somit ist die Regeneration der vorbeschriebenen epithelialen, selektiven Permeabilitätsbarriere zur Wiederherstellung der Barrierefunktion von Epithelgeweben auf die körpereigene Vitalität angewiesen. Allerdings ist diese nach einem Krankheitsverlauf und aufgrund potentiell auftretender Nebenwirkungen häufig herabgesetzt und der Körper kann nur sehr langsam oder ist gar nicht in der Lage die Barriere von Epithelgeweben aufrechtzuerhalten oder wiederherzustellen.

Bislang wurde kein detaillierter Wirkmechanismus beschrieben, der bei gesundheitlichen Beeinträchtigungen durch Einwirkung von biobasierten Noxen auf die Qualität der epithelialen Barriere (insbes. selektive Permeabilitätsbarriere oder Barrierefunktion in Epithelgeweben) einen positiven Einfluss aufweist. Für die vorgenannten Stoffklasse ebenfalls nicht.

Daher ist es Aufgabe der vorliegenden Erfindung eine Verbindung oder ein Gemisch bereitzustellen, das geeignet ist Defekte der Barrierefunktion in Epithelgeweben zu minimieren, zu verhindern oder zu behandeln. Es ist ebenfalls Aufgabe der vorliegenden Erfindung eine Verbindung oder ein Gemisch bereitzustellen, das Epithelgewebe vor negativen Einflüssen durch biobasierte Noxen schützt. Dabei soll die Verbindung oder das Gemisch die Barriere stabilisieren, sodass ein unkontrolliertes Eindringen biobasierter Noxen gehemmt oder verhindert wird. Es soll eine Verbindung oder ein Gemisch zur Verwendung bei der Prävention oder Behandlung von durch biobasierte Noxen verursachter oder mit mindestens einer biobasierten Noxe assoziierter Beeinträchtigung der Barrierefunktion von Epithelgeweben bereitgestellt werden. Es ist Aufgabe der vorliegenden Erfindung die Stabilität, die Regeneration und die Funktion der epithelialen Barriere in äußeren Epithelgeweben umfassend die Oberhaut, in Übergangsepithelgeweben umfassend die Mund- und Nasenhöhle und/oder in inneren Epithelgeweben umfassend die unteren Atemwege und das innere Endothel zu schützen und/oder zu stabilisieren. Weiter soll eine Verbindung oder ein Gemisch bereitgestellt werden, das durch biobasierte Noxen verursachte Effloreszenzen, Neurodermitis, Entzündungen der Haut, des Auges, der Mund-/Nasenschleimhaut, Atemwegserkrankungen, Flüssigkeitsverlust, Austrocknung der (Schleim-)haut, Bindehaut, Hornhaut und/oder allergische Reaktionen von Epithelgeweben verhindert oder reduziert. Es ist ebenfalls Aufgabe der vorliegenden Erfindung eine Verbindung oder ein Gemisch zur Verwendung bei der Prävention oder Behandlung der vorgenannten gesundheitlichen Beeinträchtigungen und Krankheiten umfassend mindestens einen Barrieredefekt in mindestens einem Epithelgewebe bereitzustellen. Weiterhin soll eine Zusammensetzung enthaltend die vorbeschriebene Verbindung oder das Gemisch zur Verwendung bei der Prävention oder Behandlung von Effloreszenzen der menschlichen oder tierischen Haut und Schleimhaut bereitgestellt werden. Eine andere Aufgabe der vorliegenden Erfindung ist es kosmetische Formulierungen und Formulierung für Medizinprodukte und Arzneimittel enthaltenden das vorbeschrieben Solut und/oder die vorbeschriebene Zusammensetzung umfassend die mindestens eine Verbindung zur oralen, nasalen oder topischen Verabreichung bereitzustellen. Es ist Aufgabe der Erfindung Kosmetikprodukte, Medizinprodukte und Arzneimittel zur Prävention oder Behandlung von mit mindestens einer biobasierten Noxe assoziierter Barrieredefekte von Epithelgeweben, insbesondere von Krankheiten umfassend mindestens einen Barrieredefekt in mindestens einer Zelllage von mindestens einen Epithelgewebe, bereitzustellen.

Überraschend wurde nun festgestellt, dass das kompatible Solute Ectoin und seine Derivate eine solche Wirksamkeit aufweisen.

Daher ist ein Gegenstand der vorliegenden Erfindung ein kompatibles Solut oder Solutgemisch zur Verwendung bei der Prävention oder Behandlung von mit mindestens einer biobasierten Noxe assoziierter Barrieredefekte von Epithelgeweben, vorzugsweise gestörte und verminderte selektive Permeabilitätsbarriere, insbesondere von Krankheiten umfassend mindestens einen Barrieredefekt in mindestens einer Zelllage von mindestens einem Epithelgewebe, enthaltend mindestens eine Verbindung ausgewählt aus Verbindungen der Formel I, der Formel II, den physiologisch verträglichen Salzen der Formel I und Formel II, den stereoisomeren Formen der Verbindungen der Formel I, Formel II und der physiologisch verträglichen Salze der stereoisomeren Formen oder einem Gemisch aus mindestens zwei der vorgenannten Verbindungen, wobei in Formel I 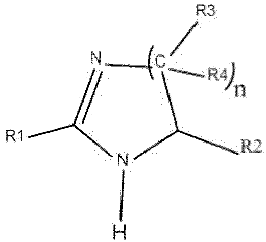 und in Formel II 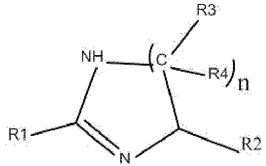
R1 = H oder Alkyl,
R2 = H, COOH, COO-Alkyl oder CO-NH-R5,
R3 und R4 jeweils unabhängig voneinander H oder OH,
R5 = H, Alkyl, ein Aminosäurereste, Dipeptidreste oder Tripeptidreste
n = 1, 2 oder 3, und Alkyl = einen Alkylrest mit C₁-C₄ Kohlenstoffatomen bedeuten.

Alkyl im Sinne der Erfindung umfasst lineare, zyklische und verzweigte Alkylreste umfassend Methyl (-CH3), Ethyl (-C2H5), Propyl (-CH2CH2CH3 oder -CH(CH3)₂) und Butyl (-CH2CH2CH2CH3, H3C(CH)CH2CH3, -CH2CH(CH3)₂ und C(CH3)₃). Bevorzugt sind lineare Alkylgruppen und besonders bevorzugt ist die Methylgruppe.

Aminosäurereste leiten sich von den entsprechenden Aminosäuren und ihren stereoisomeren Formen ab, wie L- und D- Formen, und umfassen die Aminosäuren Alanin, ss-Alanin, Arginin, Asparagin, Asparaginsäure, Cystein, Glutamin, Glutaminsäure, Glycin, Histidin, Isoleucin, Leucin, Lysin, Methionin, Phenylalanin, Serin, Threonin, Tryptophan, Tyrosin, Valin, y-Aminobutyrat, Ne-Acetyllysin, NS-Acetytornithin, Ny-Acetyldiaminobutyrat und Na-Acetyldiaminobutyrat. Bevorzugt werden L-Aminosäuren, wie L-Cystein, L-Valin, L-Arginin, L-Asparagin, L-Histidin, L-Tryptophan, L-Phenylalanin und L-Lysin. Die Aminosäurereste der Aminosäuren Alanin, L-Alanin, Asparagin, Asparaginsäure, Glutamin, Glutaminsäure, Glycin, Serin, Threonin, Valin, y-Aminobutyrat, N-Acetyllysin, N-Acetylornithin, Ny-Acetyldiaminobutyrat und Na-Acetyldiaminobutyrat sind besonders bevorzugt.

Dipeptidreste sind aus zwei Aminosäuren aufgebaut und umfassen lineare und zyklische Dipeptidreste, wobei lineare Dipeptidreste eine und zyklische Dipeptidreste zwei Peptidbindung aufweisen. Tripeptidreste sind aus drei Aminosäuren aufgebaut und umfassen lineare und zyklische Tripeptidreste, die bei linearer Struktur drei oder bei zyklischer Struktur vier Peptidbindungen aufweisen. Eine Peptidbindung ist eine Amidbindung (-CO-NH-) zwischen dem Stickstoff-Atom der Amin-Gruppe einer ersten Aminosäure und einem Sauerstoff-Atom der Carboxy-Gruppe einer zweiten Aminosäure. Bevorzugte Dipeptidreste und Tripeptidreste bestehen aus den vorgenannten Aminosäuren und besonders bevorzugt aus den vorbeschriebenen besonders bevorzugten Aminosäuren.

Physiologische Salze der Verbindung der Formel I und der Verbindung der Formel II umfassen Alkali-, Erdalkali- oder Ammoniumsalze, wie Na-, K-, Mg- oder Ca-Salze, sowie Salze abgeleitet von organischen Basen, z.B. aliphatische oder aromatische Amine, wie Triethylamin oder Tris-(2-hydroxy-ethyl)-amin. Bevorzugte physiologisch verträgliche Salze der Verbindungen der Formel I und der Formel II werden durch Umsetzung mit anorganischen Säuren erzielt, wie Salzsäure, Schwefelsäure und Phosphorsäure, oder mit organischen Carbon- oder Sulfonsäuren, wie Essigsäure, Citronensäure, Benzoesäure, Maleinsäure, Fumarsäure, Weinsäure und p-Toluolsulfonsäure.

Ebenfalls Gegenstand der vorliegenden Erfindung ist die Verwendung der Verbindungen der Formel I und der Formel II, den physiologisch verträglichen Salzen der Formel I und Formel II, den stereoisomeren Formen der Verbindungen der Formel I, Formel II und der physiologisch verträglichen Salze der stereoisomeren Formen oder eines Gemisches, sowie der hierin beschriebenen bevorzugten Ausführungsformen, zur Herstellung eines Kosmetikproduktes, Medizinproduktes, in-vitro-Diagnostikproduktes (IVD), Arzneimittels und/oder eines Zusatzes oder Bestandteils zu einem der genannten Erzeugnisse zur Prävention oder Behandlung von mit mindestens einer biobasierter Noxe assoziierter Barrieredefekte von Epithelgeweben, insbesondere von Krankheiten umfassend mindestens einen Barrieredefekt in mindestens einer Zelllage mindestens eines Epithelgewebes. Vorzugsweise Krankheiten mit einer veränderten selektiven Permeabilitätsbarriere umfassend eine reduzierte Claudin-Expression.

Bevorzugte Verbindungen der Formel I und der Formel II sind Verbindungen, worin R1 ein Wasserstoff-Atom oder ein Methylrest (CH3) ist, R2 ein Wasserstoff-Atom oder ein COOH ist, R3 und R4 jeweils unabhängig voneinander ein Wasserstoff-Atom oder ein OH und n gleich 2 ist. Insbesondere bevorzugte Verbindungen gemäß den vorgenannten Definitionen sind 1,4,5,6-Tetrahydro-2-methyl-4-pyrimidincarbonsäure (Ectoin) und 1,4,5,6-Tetrahydro-5-hydroxy-2-methyl-4-pyrimidincarbonsäure (Hydroxyectoin) sowie die physiologisch verträglichen Salze und stereoisomeren Formen der vorgenannten Verbindungen. Bevorzugt sind die Verbindungen der Formel I und der Formel II (S)-1,4,5,6-Tetrahydro-2-methyl-4-pyrimidin-carbonsäure (S-Ectoin) und (S,S)-1,4,5,6-Tetrahydro-5-hydroxy-2-methyl-4-pyrimidincarbonsäure ((S,S)-Hydroxyectoin).

Zur Verwendung bei der Prävention oder Behandlung von durch biobasierte Noxen verursachte Barrieredefekte von Epithelgeweben, insbesondere Krankheiten umfassend mindestens einen Barrieredefekt in mindestens einem Epithelgewebe, sind weiter bevorzugt Verbindungen der Formel I und der Formel II, worin R1 ein Wasserstoff-Atom oder ein Methylrest (CH3) ist, R2 ein Wasserstoff-Atom oder ein COOH ist, R3 und R4 jeweils unabhängig voneinander ein Wasserstoff-Atom oder ein OH und n gleich 3 oder 4 ist. Insbesondere bevorzugte Verbindungen gemäß den vorgenannten Definitionen sind (S)-4,5,6,7-Tetrahydro-2-methyl-1H-[1,3]-diazepin-4-carbonsäure (Homoectoin) mit n gleich 3 und 3,4,5,6,7,8-Hexahydro-2-methyl-1,3-diazocin-4-carbonsäure (HHMDCA) mit n gleich 4, sowie die physiologisch verträglichen Salze und stereoisomeren Formen der vorgenannten Verbindungen.

Die vorbeschriebenen Verbindungen können als optische Isomere, Diastereomere, Racemate, Zwitterionen, Kationen oder als ein Gemisch aus mindestens zwei der vorgenannten Formen vorliegen. Isomere umfassen (R,R)-, (R,S)-, (S,S)- und (S,R)-Konfigurationen der vorgenannten Verbindungen. Neben Isomere sind Diastereomere, Racemate, Zwitterionen, Kationen und Gemisch der vorgenannten Verbindungen ebenfalls Gegenstand der Erfindung. Derivatisierungen können mit Hydroxy-, Sulfsonsäure-, Carboxysäurederivate, wie Amide, Ester usw., Carbonyl, Ether, Alkoxy- und Dydroxylgruppen erfolgen. Ein mögliches Derivat, ohne sich darauf zu beschränken, ist (S,S)-alpha-Amino-beta-Hydroxyectoin.

Die Terminologie "Solut" ist synonym für "kompatibles Solut" und "Gemisch synonym für "Solutgemisch" zu verstehen. "Solutgemisch" bezieht sich stets auf eine Mischung aus mindestens zwei Solute der Formel I und/oder Formel II. "Ectoin", "Hydroxyectoin" und "Homoectoin" umfasst stets alle stereoisomere Formen der jeweiligen Verbindung. Spezifische Isomere werden als solche gekennzeichnet.

In einer erfindungsgemäßen Ausführungsform der Verwendung des kompatibles Soluts oder Solutgemisch wird die mindestens eine biobasierte Noxe ausgewählt aus tierischen Noxen, pflanzlichen Noxen, Noxen der Insekten und der Schädlinge, mikrobiellen Noxen, Umweltnoxen, Lebensmittelnoxen, jeweils Bestandteilen und/oder Verbindungen der vorgenannten Noxen und/oder Kombinationen aus mindestens zwei der biobasierten Noxen.

Biobasierte Noxen im Sinne der Erfindung sind ausschließlich natürlichen Ursprungs, aus der Natur stammend und biogenen Ursprungs. Dies bezieht ebenfalls Wind und Wetter bedingte Temperaturen und Luftfeuchtigkeitsanteile in der Umwelt ein. Im Sinne der Erfindung sind biobasierte Noxen vorzugsweise biobasierte Noxen, die mit dem menschlichen oder tierischen Organismus zumindest über das äußere Epithelgewebe in Kontakt treten. Bevorzugt sind biobasierte Noxen peptidbasiert. Wirken biobasierte Noxen ausschließlich über den äußeren Kontakt mit dem äußeren Epithelgewebe, so sind diese Kontaktnoxen. Andere Noxen wirken ebenfalls oder erst nach Aufnahme über das äußere Epithelgewebe und/oder über die Schleimhaut (Mund, Nase, Augen, Magen-Darm). Es können biobasierte belebte Noxen (Pflanzen, Insekten, Schädlinge, Bakterien, Viren, Pilze, Hefen jeweils reproduktionsfähig) von biobasierten unbelebten Noxen unterschieden werden. Letztgenannte umfassen tierische, mikrobielle und pflanzliche Absonderungen einschließlich Toxine, Verbindungen, Kot, Sekrete, Exkrete, Lebensmittelbestandteile und physikalische Noxen wie Wind, Temperatur, Luftfeuchtigkeit und Sonne (photodynamische Lichteinwirkung). Biobasierte Noxen umfassen ebenfalls biobasierte Haptene, die erst in Kombination mit einem Protein als Noxe im menschlichen Organismus wirken können. Das Protein kann eine weitere erfindungsgemäße biobasierte Noxe sein oder ein körpereigenes Protein des betroffenen Menschen. Bevorzugt sind biobasierte Noxen, insbesondere biobasierte Kontaktnoxen, peptidbasiert.

Bevorzugt wird die mindestens eine Noxe oder eine Kombination von mindestens zwei biobasierten Noxen ausgewählt aus mindestens einer der Gruppen
a) tierische Noxen umfassend Haustiere, Katze, Hund, Schwein, Nagetiere, Nutztiere, Schwein, Ziege, Absonderungen der vorgenannten Tiere, Tierepithelien und/oder Tierhaare,
b) pflanzliche Noxen umfassend Blütenpollen, Fruchtorgane, Säfte, Sekrete, Gifte, Harze, Duftstoffe, Aromastoffe, Toxine, Brennhaare, Haken, Nadeln, Dornen, Bestandteile und/oder Verbindungen pflanzlicher Noxen,
c) Noxen der Insekten und der Schädlinge umfassend Milben, Hausstaubmilben, Absonderungen von Insekten, Schädlingen und/oder Parasiten, Bienenharze, Bienengifte, Wespengifte, Spinnengifte, Schädlingsbisse, Mücken-, Bremsen- und Ameisenstiche oder jeweils -bisse, Bestandteile, wie z.B. Stachel, Gifthaare, Wiederhaken, und/oder Verbindungen von Insekten oder Schädlingen,
d) mikrobielle Noxen umfassend Mikroorganismen, Pilze, Hefen, Malassezia-Spezies, Bakterien, Staphyloccocus aureus, Schimmelsporen, Schimmelpilze, bakterielle Toxine, Delta-Toxin, Antibiotika, Viren, Mykotoxine, Bestandteile und/oder Verbindungen der Mikroorganismen,
f) Umweltnoxen wie photodynamische Lichteinwirkung, Wind, saisonale Temperaturschwankungen,
e) Lebensmittelnoxen umfassend Nüsse, Erdnüsse, Haselnüsse, Wein, Kuhmilch, Weizen, Soja, Hühnerei, Eiweiß, Fisch, Schalentiere, Krebstiere, Weichtiere, rohes Gemüse, rohes Obst, Bestandteile und/oder Verbindungen von Lebensmitteln, und/oder
f) insbesondere humane Noxen umfassend körpereigene Bestandteile/Verbindungen, Schweiß, Proteine und/oder Aminosäuren.

Die jeweiligen Noxen können selbst einen negativen Einfluss auf die Barrierefunktion von Epithelgeweben (Tabelle 2), insbesondere auf die selektive Permeabilitätsbarriere, haben oder erst durch eine Kombination aus mindestens zwei der vorgenannten Noxen einen negativen bis schädigenden Einfluss auf Epithelgeweben haben. Insbesondere können auf primärere Schädigungen der Barrierefunktion (z.B. mechanische Läsionen durch Umweltnoxen, wie hohe Temperaturen, starke Kälte und/oder Wind) sekundäre, teilweise stärkere, Barrieredefekte (z.B. durch infektiöse Noxen z.B. der Mikroorganismen ausgelöste Infektionen und durch nicht-infektiöse Noxen ausgelöste Allergien) folgen.

**Tabelle 1: Biobasierte Noxen im Sinne der Erfindung**

| **Gruppe** | **Einzelne Noxen** |
|---|---|
| a) Tierische Noxen | Haustiere, wie Katze, Hund, Schwein, Nagetiere, etc.; Nutztiere, Schwein, Ziege, Gans etc.; Absonderungen der vorgenannten Tiere (Kot, Urin, Schweiß, Körperflüssigkeiten), Tierepithelien, Tierhaare (Anhangsgebilde, Hautschuppen) Meerestiere: Quallen, Seeigel, Stechrochen, Kegelschnecken und Petermännchen; Schwämme (Kieselsäurenadeln), Seeanemonen; Nesseltiere, wie Quallen, Scheibe-/Schirmquallen, Ohrenqualle und Würfelqualle sowie Seewespen und jeweils ihre Gifte (Tentakel-/ Fangarmkontakt), Nesselkapseln, Dornen, Feuerqualle, Leuchtqualle und Kompassqualle; Algen und ihre Absonderungen und Toxine |
| b) Pflanzliche Noxen | Blütenpollen, Fruchtorgane, Säfte, Sekrete, Gifte, Harze, Naturkautschuk, Latex, Duft- und Aromastoffe, Toxine, Brennnessel und andere reizende Pflanzen (z.B. Arnika, Beifuß, Kamille und Schafgarbe), Harze, Brennhaare, Haken, Nadeln, Dornen, Bestandteile und/oder Verbindungen pflanzlicher Noxen; Pflanzliche Stoffe, z.B. Perubalsam (Balsamum peruvianum), Baummoss (Evernia |
| | furfuracea), Eukalyptus, usw.; Wollwachsalkohole, Lanolin, Arnika, Kamille, Bienenharz, Ringelblume |
| | Gräser-, Birke-, Haselpollen sind häufige Auslöser für Allergien gegen: Steinobst (Apfel, Birne), Kernobst (Pflaume, Kirsche, Pfirsich), Erd-/ Haselnuss, Paranuss, Walnuss, Mandel, Sellerie, Karotte, Kiwi, Gewürze, wie Anis, Curry; Gräserpollen sind häufige Auslöser für Allergien gegen: Getreidemehl, Erdnuss, Soja (-bohne, -mehl, milch); |
| | Kräuterpollen sind häufige Auslöser für Allergien gegen: Sellerie, Karotte, Fenchel, Knoblauch; Kamille, Petersilie; Sonnenblumenkerne; Gewürze, wie Kümmel, Curry, Paprika, Anis, Pfeffer, Muskat, Zimt, Ingwer, Koriander |
| c) Insekten, Schädlinge | Milben, Hausstaubmilben und Milbenabsonderungen; Ameise, Spinne, Würmer, Läuse, etc. Schädlingsbisse, Schädlingsflüssigkeiten, Ameisengifte, Spinnengifte, Bienen-/Wespengifte (Allergene: Glycoproteine, Phospholipase A1 und A2, Hyaluronidase, Melithin, Apamin, MCD-Peptid 401, Antigen 5), Spinnen-, Mücken-, Bremsen- und Ameisenstiche- und/oder jeweils -bisse, Bienenharz; Schädlings- und Insektenbestandteile, wie z.B. Stachel, Gifthaare, Wiederhaken |
| d) Mikrobielle Noxen | Mikroorganismen, Pilze, Hefen, infektiöse Mikroorganismen, Malassezia-Spezies, Bakterien, Staphyloccocus aureus, Schimmelsporen, Schimmelpilze, bakterielle Toxine, Delta-Toxin, Antibiotika, Viren, Mykotoxine, Bestandteile und/oder Verbindungen der Mikroorganismen, Exotoxine, Endtoxine, Corynebacterium diphtheriae |
| e) Lebensmittelnoxen | Nüsse, Erdnüsse, Haselnüsse, Wein, Kuhmilch, Weizen, Soja, Hühnerei, Eiweiß, Fisch, Schalentiere, Krebstiere, Weichtiere, rohes Gemüse, rohes Obst, Bestandteile und/oder Verbindungen von Lebensmitteln |

In einer weiteren Ausführungsform der erfindungsgemäßen Verwendung des mindestens einen kompatiblen Soluts oder Solutgemisches ist die mindestens eine biobasierte Noxe peptidbasiert. Peptidbasiert im Sinne der Erfindung bedeutet, dass die biobasierte Noxe eine Verbindung ist oder mindestens eine Verbindung umfasst, welche mindestens eine Peptidbindung zwischen mindestens zwei Aminosäuren umfasst.

In weiteren Ausführungsform der erfindungsgemäßen Verwendung des mindestens einen kompatiblen Soluts oder Solutgemisches umfassen Epithelgewebe, insbesondere Oberflächenepithel als Grenze zwischen inneren und äußeren Oberflächen sowie zwischen funktionellen Einheiten,
- äußere Epithelgewebe umfassend Haut, Oberhaut, Kopfhaut, Epidermis, Nagelbett, Nagelplatte (Eponychium), Hornhaut und Bindehaut, und insbesondere Schleimhaut, des Auges, Außenohr, äußerer Gehörgang und Lippen,
- Übergangsepithelgewebe umfassend Mundhöhle, Mundschleimhaut, Zahnfleisch, Zunge, Zungenschleimhaut, obere Atemwege, Nasenhöhle, Nasennebenhöhlen, Nasenschleimhaut, Stimmlappen, Rachen und Genitalien und/oder
- innere Epithelgewebe umfassend untere Atemwege, Luftröhre, Bronchien, Bronchialbaum, Lungen, inneres Endothelgewebe, kontinuierliches Endothel, insbesondere kontinuierliches Endothel von Lunge und Herz, Endothel von Herz-, Blut-Lymphgefäßen, Speiseröhre, Magenschleimhaut und/oder Dünn-/Darmschleimhaut.

Epithelgewebe werden histologisch nach der Anzahl der Zellschichten in ein einschichtiges, mehrschichtiges und mehrreihiges Epithel unterteilt. Ferner wird das Epithel nach der Form der Zellen eingeteilt in ein flaches Epithel, isoprismatisches oder kubisches Epithel und hochprismatisches bzw. zylindrisches Epithel. Der Grad der Keratinisierung wird als verhornt oder unverhornt beschrieben. Abhängig von der Lokalisation und Funktion des Epithels weist das Epithel eine charakteristische Histologie auf. Nachfolgend wird das Vorkommen der im Sinne der Erfindung umfassten Epithelgewebe zusammengefasst, sodass wenn im Sinne der Erfindung von Epithelgewebe gesprochen wird, die nachfolgende Zusammenstellung maßgebend ist, es sei denn es wird explizit auf eine besondere Ausführungsform verwiesen.

**Tabelle 2: Epithelgewebe im Sinne der Erfindung**

| **Epithelgewebe** | **Vorkommen** |
|---|---|
| Einschichtiges Plattenepithel | Seröse Häute umfassend Pleura, Lungenfell, Herzbeutel, Scheidenhaut des Hodens; Alveolenepithel, Endothel, Lunge (Endothel), Endothel von Herz, Blut- und Lymphgefäßen; Zungenschleimhaut (Zungenunterseite ) |
| Einschichtiges hochprismatisches Epithel | Epithel von Magen, Dünn- und Dickdarm; Magenschleimhaut, Darmschleimhaut |
| Zweireihiges Epithel | Speicheldrüse, Mundhöhle, Tränenkanal |
| Mehrreihiges hochprimsatisches Epithel | Nasenhöhle, Schlund (Rachen), Kehlkopf, Atemwege, Bronchialbaum, Ohrtrompete, Harnröhre, |
| Mehrschichtiges unverhorntes Epithel | Vorderes Hornhautepithel (Auge), Stimmfalte, Mundhöhle, Zahnfleisch (innere Saumepithel, das den Zahnhals umschließt), Rachen, Speiseröhre, After, Vagina, Zungenschleimhaut (Zungenoberseite); Bindehaut (Konjunktiva) des Auges, Kornea |
| Mehrschichtiges verhorntes Epithel | Epidermis (Stratum basale, Stratum spinosum, Stratum granulosum), Nasenvorhof, äußerer Gehörgang, Zahnfleisch (äußeres Saumepithel zur Mundhöhle), |

Die Epithelgewebe der Tabelle 2 umfassen als interzellulären Verbindungen Tight junction, Adherens junction und/oder Desmosomen. Dabei wird durch die Tight junctions die eigentliche parazelluläre Barrierefunktion der Epithelgewebe, insbesondere selektive Permeabilitätsbarriere, erzielt, wodurch Transzytose ermöglicht und gleichzeitig der Verlust von Körperflüssigkeiten verhindert wird. Ferner wird das parazelluläre Eindringen von Molekülen, Ionen, Antigenen, peptidbasierten Noxen und Mikroorganismen verhindert. Eine wichtige Funktion ist die selektive Resorption und Sekretion von Nährstoffen, Elektrolyten und Wasser. Durch die Adherens junctions und Desmosomen werden die Zellen verbunden und dadurch mechanisch stabilisiert. Tight junction tragen neben ihrer Funktion bei der selektiven Permeabilitätsbarriere auch zur mechanischen Stabilisierung des Epithelgewebes bei. Daher liegt der Fokus der vorliegenden Erfindung auf der Untersuchung der Permeabilitätseigenschaften und Beeinflussung der Barrierefunktion von Epithelgeweben.

Daher ist ein weiterer Gegenstand der vorliegenden Erfindung ein kompatibles Solut oder Solutgemisch zur erfindungsgemäßen Verwendung, wie beschrieben, wobei der mindestens eine Barrieredefekt von mindestens einem Epithelgewebe eine gestörte interzellulare Zellstruktur in mindestens einer Zelllage der Epithelzellen vom äußeren Epithelgewebe, Übergangsepithelgewebe und/oder inneren Epithelgewebe aufweist. Vorzugsweise umfasst das mindestens eine Epithelgewebe als interzelluläre Verbindungen Tight junction, Adherens junction und/oder Desmosomen auf. Defekte der vorgenannten Verbindungen führen zur strukturellen Schwächung und damit zu einer gestörten und verminderten selektiven Permeabilitätsbarriere.

Tight junction sind aufgrund ihrer vielseitigen Aufgaben die wichtigsten Komponenten in der Aufrechterhaltung der Barrierefunktion von Epithelgeweben. Sie umschließen die Epithelzellen ringförmig am apikalen Ende als intramembranöse, kontinuierliche Struktur. Betrachtet man die räumliche Anordnung der interzellulären Verbindungen in Epithelgeweben, so sind Tight junction in allen Epithelgeweben am weitesten apikal angeordnet, gefolgt von Adherens junction, Desmosomen und Gab junction als die basal angeordnete interzelluläre Verbindung. Somit wirken biobasierte Noxen vorrangig auf Tight junction ein. Erst wenn diese geschädigt oder defekt sind und die biobasierte Noxe aufgrund einer fehlenden Permeabilitätsbarriere eindringen kann, wirken die biobasierte Noxe auf die Adherens junction usw.. Weisen die vorgenannten Epithelgeweben bzw. Epithelzellen Defekte in der Barriere (synonym selektive Permeabilitätsbarriere) auf, so ist primär von einer Schädigung oder Verlust von Tight junction auszugehen (siehe Beispiele 1 bis 3).

Daher ist ein weiterer Gegenstand der vorliegenden Erfindung ein kompatibles Solut oder Solutgemisch zur erfindungsgemäßen Verwendung, wie beschrieben, wobei der mindestens eine Barrieredefekt des mindestens einen Epithelgewebes eine Schädigung der Tight junction in mindestens einer Zelllage auf. Insbesondere führt eine Schädigung der Tight junction zu einer gestörten und verminderten selektiven Permeabilitätsbarriere, insbesondere im interzellularen Raum.

In einer weiteren Ausführungsform der vorliegenden Erfindung bei der Verwendung des mindestens einen kompatiblen Soluts oder Solutgemisches weist der mindestens eine Barrieredefekt in mindestens einem Epithelgewebe eine gestörte interzellulare Zellstruktur, insbesondere eine erhöhte Permeabilität, in mindestens einer Zelllage der Epithelzellen vom äußeren Epithelgewebe, Übergangsepithelgewebe und/oder inneren Epithelgewebe auf. Vorzugsweise umfasst der mindestens eine Barrieredefekt eine Schädigung der Tight junction in mindestens einer Zelllage des mindestens einen Epithelgewebes. Schädigungen der Tight junction können eine geringere Proteinstabilität, Modulation der Proteine, insbesondere der Occludin- und/oder Claudin-Proteinfamilie, teilweise Degradation oder Brüche der transzellulären Schleifen der Tight junction Proteine sein, wodurch die Zellstruktur beeinträchtigt und durchlässiger wird. Die Beeinträchtigung der Tight junction Proteine führt zur Störung und Herabsenkung der selektiven Permeabilitätsbarriere von Epithelgeben, wodurch wiederum ein erhöhter parazellulärer Fluss von biobasierten Noxen resultiert (Beispiel 2).

Daher ist in einer weiteren Ausführungsform der vorliegenden Erfindung bei der Verwendung des mindestens einen kompatiblen Soluts oder Solutgemisches der mindestens eine Barrieredefekt des mindestens einen Epithelgewebes eine gestörte und verminderte selektive Permeabilitätsbarriere. Eine gestörte bzw. geschädigte selektive Permeabilitätsbarriere kann anhand eines reduzierten transepithelialen elektrischen Widerstand (TEER) festgestellt werden, wie in Beispiel 1 gezeigt, und anhand einer geringeren Expression von mindestens einem Protein der Claudin-Proteinfamilie, wie in Beispiel 2 gezeigt.

Die mindestens eine biobasierte Noxe (Tabelle 1) kann Ursache für die Störung der Barrierefunktion sein und/oder kann bei bereits prädisponierten Epithelgeweben mit geschwächter Barrierefunktion z.B. bei äußeren Epithelgeweben, wie der Epidermis, Horn- und/oder Bindehaut, zu stärkeren Barrieredefekten in Epithelgeweben und daraus resultierenden Krankheiten führen. Vorzugsweise Claudin-assoziierte Erkrankungen mit einer veränderten selektiven Permeabilitätsbarriere umfassend eine reduzierte Claudin-Expression. Claudin-assoziierte Erkrankungen umfassen inflammatorische Darmerkrankungen, Nierenerkrankungen, bakterielle und virale Infektionen sowie Hauterkrankungen. Insbesondere bei Psoriasis, Dermatitis und atopischer Dermatitis und verwandter Hauterkrankungen mit einer Barrierestörung weisen die Epithelgewebe insbesondere in der frühen Phase eine verminderte selektive Permeabilitätsbarriere auf, unter anderem begründend auf einer herunterregulierten Claudin-Expression

Wie bereits Eingangs erläutert ist die Regeneration der vorbeschriebenen epithelialen, selektiven Permeabilitätsbarriere zur Wiederherstellung der Barrierefunktion von Epithelgeweben auf die körpereigene Vitalität angewiesen. Allerdings ist diese nach einem Krankheitsverlauf und aufgrund potentiell auftretender Nebenwirkungen häufig herabgesetzt und der Körper kann nur sehr langsam oder ist gar nicht in der Lage die Barriere von Epithelgeweben wiederherzustellen oder aufrechtzuerhalten. Die hier beschriebenen Versuche zeigen, dass Ectoin den Körper bei der Regeneration der selektiven Permeabilitätsbarriere unterstützten kann, was an der Erhöhung der Claudin-Expression und einem erhöhten TEER nachgewiesen wurde (Beispiele 1 bis 3).

In einer weiteren Ausführungsform der erfindungsgemäßen Verwendung mindestens eines kompatiblen Soluts oder Solutgemisches weist das mindestens eine Epithelgewebe mit mindestens einem Barrieredefekt, vorzugsweise in mindestens einer Zelllage, im Vergleich zu nicht geschädigten Epithelgeweben, einen reduzierten transepithelialen elektrischen Widerstand (TEER) auf, welcher in [Ohm] gemessen wird, wie in Beispiel 1 erläutert. Insbesondere wird erfindungsgemäß durch Einwirkung mindestens eines Soluts der TEER der vorgenannten Epithelgewebe erhöht und/oder stabilisiert. Bei einer intakten selektiven Permeabilitätsbarriere, wird die freie Diffusion durch die Zellzwischenräume (parazellulärer Weg) durch die selektive parazelluläre Permeabilitätsbarriere eingeschränkt, deren Dichtheit gewebsspezifisch ist und typischerweise durch den transepithelialen elektrischen Widerstand (TEER) beschrieben wird. Die Dichtheit der selektiven parazellulären Permeabilitätsbarriere wird durch Tight junction bewirkt. Adherens junction sowie Desmosomen verbinden die Zellen mechanisch miteinander.

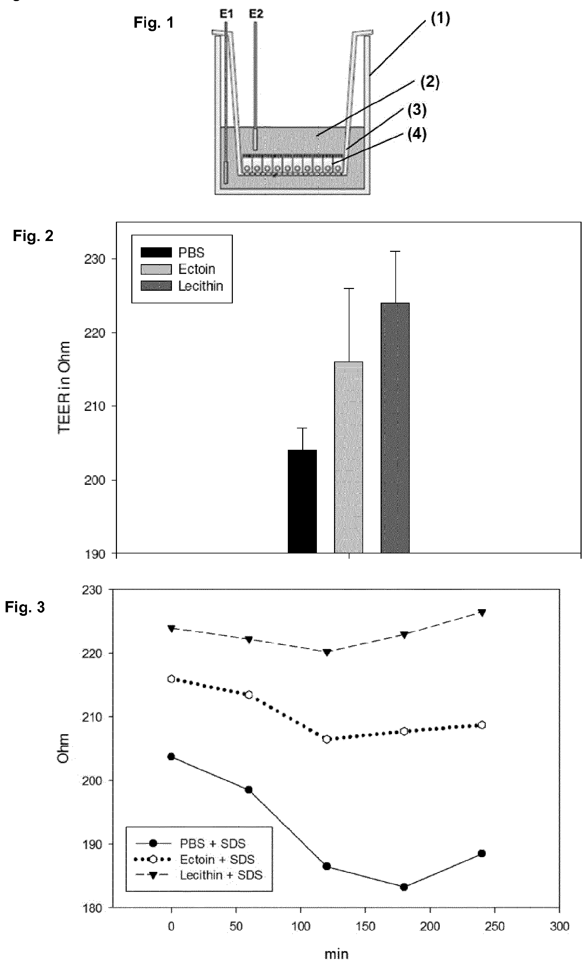

In Beispiel 1 wurden repräsentativ für alle erfindungsgemäßen Epithelgewebe (Tabelle 2) Epithelzellen der Mundschleimhaut (TR146-Zellen = Übergangsepithelgewebe), renale Epithelzellen aus dem Schwein (LLC-PK1 = inneres Epithelgewebe) sowie humane Keratinozyten (HaCat Zellen= äußeres Epithelgewebe) untersucht. In allen Epithelgewebearten konnte ein positiver Einfluss von Ectoin auf die Stabilisierung des transepithelialen elektrischen Widerstands (TEER) nachgewiesen werden (Fig. 3, Fig. 4, Fig. 5 und Fig. 6).

In einer weiteren Ausführungsform der erfindungsgemäßen Verwendung mindestens eines kompatiblen Soluts oder Solutgemisches weist das mindestens eine Epithelgewebe mit mindestens einem Barrieredefekt, vorzugsweise in mindestens einer Zelllage, im Vergleich zu nicht geschädigten Epithelgeweben, eine höhere Permeabilität für mindestens eine biobasierte Noxe auf, wie in Beispiel 2 anhand des Allergiepräventionsassays (APA) gezeigt. Insbesondere wird erfindungsgemäß durch Einwirkung mindestens eines Soluts die Permeabilität der vorgenannten Epithelgewebe reduziert. In Beispiel 2 wurden repräsentativ für alle erfindungsgemäßen Epithelgewebe (Tabelle 2) humane Epithelzellen der Mundschleimhaut sowie der Nasenschleimhaut (TR146-/RPMI-2650-Zellen = Übergangsepithelgewebe), bronchiale Epithelzellen aus der Ratte (RLE = inneres Epithelgewebe) sowie humane Keratinozyten und korneale Epithelzellen aus dem Kaninchen (HaCat-/SIRC-Zellen = äußeres Epithelgewebe) untersucht. In allen Epithelgewebearten konnte ein positiver Einfluss von Ectoin auf die Reduzierung der Permeabilität (APA) nachgewiesen werden (Tabelle 4).

In einer weiteren Ausführungsform der erfindungsgemäßen Verwendung mindestens eines kompatiblen Soluts oder Solutgemisches weist das mindestens eine Epithelgewebe mit mindestens einem Barrieredefekt, vorzugsweise in mindestens einer Zelllage, im Vergleich zu nicht geschädigten Epithelgeweben, eine geringere Expression von mindestens einem Protein der Claudin-Proteinfamilie auf, wie in Beispiel 3 gezeigt. Insbesondere wird erfindungsgemäß durch Einwirkung mindestens eines Soluts die Claudin-Expression der vorgenannten Epithelgeweben erhöht und/oder stabilisiert. In Beispiel 3 wurden repräsentativ für das äußere Epithelgewebe humane Keratinozyten (HaCat-Zellen) verwendet. Nach einem Hitze- und damit Austrocknungsstress (erfindungsg. Umweltnoxen) konnte ein positiver Einfluss von Ectoin auf die Expression von Claudin-1 nachgewiesen werden (Tabelle 5). Somit ist Ectoin in der Lage durch die Stimulierung der Expression von Proteinen der Claudin-Proteinfamilie die Bildung von Tight junction zu fördern. Damit wird die Barrierefunktion, insbesondere die selektive Permeabilitätsbarriere, stabilisiert oder in bereits geschädigten Epithelzellen wieder hergestellt.

Nicht geschädigte Epithelgewebe im Sinne der Erfindung entsprechen den Kontrollen der vorliegenden Beispiele und sind Epithelgewebe, die nicht durch Einwirkung von biobasierten Noxen beeinträchtigt wurden.

Die vorgenannten Wirksamkeiten von Ectoin zur Verwendung bei der Prävention oder Behandlung von Barrieredefekten in Epithelgeweben, insbesondere auf die Stabilisierung und/oder Wiederherstellung der selektiven Permeabilitätsbarriere (gemäß der Beispiele 1, 2 und 3), wird bevorzugt mit größer gleich 10 mM bis kleiner gleich 1 M des mindestens einen kompatiblen Soluts, vorzugsweise Ectoin, Hydroxyectoin und/oder seine Derivate erzielt, vorzugsweise größer gleich 10 mM bis kleiner gleich 750 mM, größer gleich 10 mM bis kleiner gleich 500 mM, größer gleich 25 mM bis kleiner gleich 500 mM, größer gleich 50 mM bis kleiner gleich 500 mM. Insbesondere 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 110 mM, 120 mM, 130 mM, 140 mM, 150 mM, jeweils +/- 5 mM Abweichung.

Folglich sind Ectoin und seine Derivate zur Verwendung sowohl bei der Prävention als auch bei der Behandlung von Barrieredefekten geeignet. Insbesondere von Krankheiten umfassend mindestens einen Barrieredefekt in mindestens einem Epithelgewebe, welcher mit mindestens einer, vorzugsweise peptidbasierter, biobasierte Noxe (Tabelle 1) assoziiert ist.

In einer bevorzugten Ausführungsform der vorliegenden Erfindung umfasst Epithelgewebe ausschließlich äußere Epithelgewebe umfassend Haut, Oberhaut, Kopfhaut, Epidermis, Nagelbett, Nagelplatte (Eponychium), Hornhaut und Bindehaut, und insbesondere Schleimhaut, des Auges, Außenohr, äußerer Gehörgang und Lippen, sowie Übergangsepithelgewebe umfassend Mundhöhle, Mundschleimhaut, Zahnfleisch, Zunge, Zungenschleimhaut, obere Atemwege, Nasenhöhle, Nasennebenhöhlen, Nasenschleimhaut, Stimmlappen, Rachen und Genitalien. Zur Prävention oder Behandlung von mit mindestens einer, vorzugsweise peptidbasierten, biobasierten Noxe (Tabelle 1) assoziierter Barrieredefekte, insbesondere der selektiven geschädigten Permeabilitätsbarriere, der bevorzugten Epithelgeweben wird mindestens ein kompatibles Solut oder Solutgemisch ausgewählt aus Verbindungen der Formel I und/oder der Formel II, vorzugsweise Ectoin und/oder Hydroxyectoin, verwendet.

In einer bevorzugten Ausführungsform der vorliegenden Erfindung, insbesondere der vorgenannten Auswahl, sind bevorzugt äußere Epithelgewebe und Übergangsepithelgewebe, welche jeweils Tight junction aufweisen. Insbesondere im Stratum granulosum und Stratum spinosum sind jeweils Tigt junction und Desmosomen stark vertreten und bilden die für den Menschen wichtigste Barriere für biobasierte, insbesondere peptidbasierte, Noxen. Barrieredefekte in diesem Fall weisen einen reduzierten transepithelialen elektrischen Widerstand (TEER) auf, eine höhere Permeabilität (APA) und/oder eine geringere Expression von mindestens einem Protein der Claudin-Proteinfamilie, jeweils im Vergleich zu nicht geschädigten Epithelgewebe mit einer intakten Barrierefunktion. Somit liegen im Sinne der Erfindung die Barrieredefekte an der Grenze zwischen apikaler und basolateraler Membran von Epithelzellen vor.

In einer weiteren Ausführungsform der erfindungsgemäßen Verwendung des mindestens einen kompatiblen Soluts oder Solutgemisches der Formel I und/oder der Formel II schützt und/oder stabilisiert das mindestens eine Solut oder Solutgemisch die Barrierefunktion des mindestens einen Epithelgewebes, insbesondere die selektive Permeabilitätsbarriere, hemmt und/oder vermindert zumindest die Störung der Barrierefunktion, insbesondere in mindestens einem Epithelgewebe, und/oder stellt zumindest diese teilweise wieder her (siehe oben; Beispiel 1-3).

In einer weiteren Ausführungsform der erfindungsgemäßen Verwendung des mindestens eines kompatiblen Soluts oder Solutgemisches der Formel I und/oder der Formel II zeigt das mindestens eine Solut oder Solutgemisch bei einer Konzentration größer gleich 1 mM bis kleiner gleich 1 M zumindest eine schützende Wirkung auf die Barrierefunktion von Epithelzellen der äußeren Epithelgewebe (vorzugsweise Haut, Epidermis), der Übergangsepithelgewebe (vorzugsweise Mund- und Nasenschleimhaut) und/oder der inneren Epithelgewebe (vorzugsweise Lungen-, Bronchien- und Magen-Darmepithel), hemmt die Störung der Barrierefunktion und/oder stellt diese zumindest teilweise der vorgenannten Epithelzellen wieder her.

Die eingesetzte Konzentration des mindestens einen kompatiblen Soluts, vorzugsweise Ectoin, Hydroxyectoin und/oder seine Derivate, beträgt größer gleich 1 mM bis kleiner gleich 1 M, größer gleich 5 mM, größer gleich 10 mM, größer gleich 15 mM, größer gleich 20 mM, größer gleich 25 mM, größer gleich 30 mM, größer gleich 35 mM, größer gleich 40 mM, größer gleich 45 mM, größer gleich 50 mM, größer gleich 55 mM, größer gleich 60 mM, größer gleich 65 mM, größer gleich 70 mM, größer gleich 75 mM, größer gleich 80 mM, größer gleich 85 mM, größer gleich 90 mM, größer gleich 95 mM, größer gleich 100 mM, größer gleich 110 mM, größer gleich 120 mM, größer gleich 130 mM, größer gleich 140 mM, größer gleich 150 mM, größer gleich 200 mM, größer gleich 250 mM, größer gleich 300 mM, größer gleich 350 mM, größer gleich 400 mM, größer gleich 450 mM, größer gleich 500 mM bis jeweils kleiner gleich 1 M, kleiner gleich 950 mM, kleiner gleich 900 mM, kleiner gleich 850 mM, kleiner gleich 800 mM, kleiner gleich 750 mM, kleiner gleich 700 mM, kleiner gleich 650 mM, kleiner gleich 600 mM, kleiner gleich 550 mM.

Besonders bevorzugte Konzentrationsbereiche sind größer gleich 10 mM bis kleiner gleich 1 M, größer gleich 10 mM bis kleiner gleich 750 mM, größer gleich 10 mM bis kleiner gleich 500 mM, größer gleich 25 mM bis kleiner gleich 500 mM, größer gleich 50 mM bis kleiner gleich 500 mM jeweils mindestens eines kompatiblen Soluts, vorzugsweise Ectoins, Hydroxyectoins und/oder seiner Derivate. Insbesondere 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 110 mM, 120 mM, 130 mM, 140 mM, 150 mM, jeweils +/- 5mM Abweichung.

Barrieredefekte können Ursache aber auch Folge einer Krankheit (Hauterscheinung, Hautkrankheit), Entzündungsreaktion aufgrund von Infektionen und/oder Allergien, Infektion (oberflächig, innerlich) und/oder einer Allergie sein. Dabei zeigt eine gestörte Barrierefunktion der jeweils beeinträchtigten Epithelgewebe unterschiedliche Symptome (synonym Phänotyp bzw. Phänotypisch). Diese können vereinzelt, lokal beschränkt, punktuell verteilt oder großflächig auftreten.

In einer weiteren Ausführungsform der erfindungsgemäßen Verwendung des mindestens einen kompatiblen Soluts oder Solutgemisches der Formel I und/oder der Formel II umfasst daher der mindestens eine Barrieredefekt mindestens eines Epithelgewebes phänotypisch Juckreiz, Hautverfärbungen, Hitze, Hitzeentwicklung, Fieber, Rötungen, trockene Haut (Xerodermie), ringförmige Hautveränderungen, Bläschen, Blasen, Pusteln, Papeln, Pickel, jeweils mit und ohne Eiterbildung, Abzesse, Fisteln, Ausschlag, Krusten, Erhebungen, Schwellung, Kratzer, Stiche, Verschorfung, Schuppung, Quaddeln, Angioodem-Ödem, Quincke-Ödem, Nesselsucht, Plaques, Geschwüre, Furunkel, Karbunkel, Ekzeme und/oder Orientbeulen.

Bei Hautkontakt mit tierischen Noxen verursachen Körperreaktionen Symptome, die sich durch brennenden Schmerz, Juckreiz und Schwellung der betroffenen Hautregion zeigen. Dies ist insbesondere bei Allergien auf Haustiere sowie bei Kontakt mit Meerestieren zu beobachten. Besonders bei Kontakt mit Nesseltieren kommt es je nach Umfang der Vernesselung zu starken Schmerzen, Hautausschlag und Blasenbildung, aber auch zu schweren Vergiftungssymptomen wie Erbrechen, Fieber, Bewusstseinstrübung, Durchblutungsstörungen und Herz-Kreislauf-Versagen. Schwellungen, Rötungen, Blasenbildungen und Absterben von Gewebe sind häufige Symptome bei Kontakt mit giftigen Tieren und Nesseltieren.

Der Kontakt mit Lebensmittelnoxen kann führen zu Quaddeln (Urtikaria), Nesselfieber, Rötung, Juckreiz, Quincke-Ödem, Hauterscheinungen, wie Neurodermitis und bei Neurodermitis zu Neurodermitisschüben, Urtikaria und Quinke-Ödem, Nesselsucht, und im Hals-Nasen-Ohrenbereich zu Niesattacken und Fließschnupfen.

In einer weiteren Ausführungsform der erfindungsgemäßen Verwendung des mindestens einen kompatiblen Soluts oder Solutgemisches der Formel I und/oder der Formel II umfasst der mit mindestens einer biobasierten, vorzugsweise peptidbasierten, Noxe assoziierte Barrieredefekt von Epithelgeweben der vorbeschriebenen Art, insbesondere umfassen Krankheiten mit mindestens einem Barrieredefekt, parasitäre, bakterielle und/oder virale Krankheiten, Mykosen, Läsionen, Trockenheit, Reizungen, Entzündungen, Überempfindlichkeiten und/oder allergische Reaktionen jeweils der äußeren Epithelgewebe, Übergangsepithelgewebe und/oder inneren Epithelgewebe. Vorzugsweise weisen die Barrieredefekte eine Schädigung der Tight junction (siehe oben) in mindestens einer Zelllage der Epithelgewebe auf. Allergische Reaktionen umfassen allergische Reaktionen auf die erfindungsbasierten Noxen (Tabelle 1), Kreuzallergien und Kontaktallergien, vorzugsweise der äußeren Epithelgewebe umfassend Haut, Oberhaut, Kopfhaut, Epidermis, Nagelbett, Nagelplatte (Eponychium), Hornhaut und Bindehaut, und insbesondere Schleimhaut, des Auges, Außenohr, äußerer Gehörgang und Lippen, sowie Übergangsepithelgewebe umfassend Mundhöhle, Mundschleimhaut, Zahnfleisch, Zunge, Zungenschleimhaut, obere Atemwege, Nasenhöhle, Nasennebenhöhlen, Nasenschleimhaut, Stimmlappen, Rachen und Genitalien.

Zur Prävention oder Behandlung der vorgenannten mit mindestens einer, vorzugsweise peptidbasierten, biobasierten Noxe (Tabelle 1) assoziierter Barrieredefekte, insbesondere Krankheiten, wird mindestens ein kompatibles Solut oder Solutgemisch ausgewählt aus Verbindungen der Formel I und/oder der Formel II, vorzugsweise Ectoin und/oder Hydroxyectoin, verwendet. Die beschriebenen bevorzugten Konzentrationsbereiche für das mindestens eine Solut gelten hier entsprechend.

Eine Kontaktallergie ist eine innerhalb von 48-72 Std. einsetzende Reaktion auf den Kontakt mit einem Allergen, bei dem das Allergen, insbesondere die biobasierte Noxe und biogene Arbeitsstoffe (Bsp: Latex, in der Land-, Forst- und Fischereiwirtschaft), das Epithelgewebe, vorzugsweise die Epidermis, intrudiert und/oder penetriert. Bei entsprechend disponierten Personen, basierend auf genetischen oder nicht-genetischen Faktoren, zeigen sich kontaktallergischen Reaktion anhand der bereits vorbeschriebenen Symptome.

Weiter bevorzugt ist die erfindungsgemäße Verwendung des mindestens einen kompatiblen Soluts oder Solutgemisches der Formel I und/oder der Formel II bei Barrieredefekten von bevorzugt äußeren Epithelgeweben und Übergangsepithelgeweben, welche jeweils Tight junction aufweisen. Insbesondere Barrieredefekte der Epidermis, die im Stratum granulosum und Stratum spinosum sind jeweils Tigt junction und Desmosomen vermehrt aufweist und die für den Menschen wichtigste Barriere für biobasierte, insbesondere peptidbasierte, Noxen bildet. Barrieredefekte in diesem Fall weisen einen reduzierten transepithelialen elektrischen Widerstand (TEER) auf, eine höhere Permeabilität (APA) und/oder eine geringere Expression von mindestens einem Protein der Claudin-Proteinfamilie, jeweils im Vergleich zu nicht geschädigten Epithelgeweben mit einer intakten Barrierefunktion. Die bevorzugten Konzentrationsbereiche für das mindestens eine Solut gelten hier entsprechend.

In einer weiteren Ausführungsform der erfindungsgemäßen Verwendung des mindestens einen kompatiblen Soluts oder Solutgemisches der Formel I und/oder der Formel II liegt mindestens ein Barrieredefekt bei Krankheiten vor, von jeweils
- äußeren Epithelgeweben (Tabelle 2) umfassend Krankheiten der Haut und der Unterhaut, Hautläsionen, Infektionen der Haut und/oder der Unterhaut, Mykosen, trockene Haut, insbesondere trockene Oberhaut der Extremitäten/Gliedmaßen, wie Beine, Füße, Arme, Hände, Armbeugen, Kniekehlen, Gesichts, Kopfes und des Halses, Kontaktallergien, Dermatitis, Ekzeme, Neurodermitis, Psoriasis, Urtikaria, Herpes, Lippenherpes, Krankheiten des Auges, Entzündungen der Bindehaut und/oder Hornhaut, Konjunktivitis, Keratitis, trockene Bindehaut, Krankheiten des äußeren Ohres, Entzündungen des Außenohrs und/oder des äußeren Gehörgangs, Otitis externa, physikalische Störungen und/oder Schädigungen der Hautstruktur umfassend Verletzungen, Stiche, Schnitte, Kratzer, Schürfungen, Verbrennungen und/oder Verätzungen jeweils ausgelöst durch Kontakt mit mindestens einer biobasierten, vorzugsweise peptidbasierten, Noxe,
- Übergangsepithelgeweben (Tabelle 2) umfassend allergische Reaktionen der Nasenschleimhaut, allergische Reaktionen mindestens einer Schleimhaut der Mundhöhle (Mund-, Zungen- und/oder Zahnfleischepithel), trockene Nasenschleimhaut, Krankheiten der Mundhöhle, Zysten, Phlegmone und/oder Abszesse der Mundschleimhaut, allergische Läsionen der Mundschleimhaut und/oder der Zunge, Krankheiten der oberen Atemwege, Nasennebenhöhlenentzündung, allergische Rhinitis, allergische Rhinopathie, Tonsillitis, Entzündungen und Infektionen der Schleimhaut von Mund, Zahnfleisches, Zunge, Rachen, Nase und/oder Genitalien, insbesondere Haut des Penis, der Eichel, des Skrotum, des Praeputium, Deckschicht der Eichel, Klitorisvorhaut, Klitoriseichel, äußere *und* inneren Schamlippen, physikalische Störungen und/oder Schädigungen der Hautstruktur umfassend Verletzungen, Stiche, Schnitte, Kratzer, Schürfungen, Verbrennungen und/oder Verätzungen jeweils ausgelöst durch Kontakt mit mindestens einer biobasierten, vorzugsweise peptidbasierten, Noxe, und/oder
- inneren Epithelgeweben (Tabelle 2) umfassend Krankheiten des Verdauungssystems, Entzündungen der Magenschleimhaut und/oder Dünn-/Darmschleimhaut, Morbus Crohn, Colitis ulcerosa, Divertikulose, Claudin-assoziierte Erkrankungen, Krankheiten der unteren Atemwege, Entzündungen der Lunge und/oder Bronchien und/oder Asthma.

Hautläsionen umfassen Hautrötungen (Erytheme), Hautverfärbungen, ringförmige Hautveränderungen, Bläschen oder Blasen (mit und ohne Eiterbildung), Pusteln, Pickel, Krusten, Erhebungen, Verschorfung, etc., stark juckende Quaddeln (z.B. in Form von Urtikaria), Akne, Plaques (z.B. Schuppenflechte, Psoriasis), Geschwüre, Furunkel, Karbunkel, Orientbeulen (Hautleishmaniose, Kutane Leishmaniose), Lidschwellung. Die Konjunktiva kann durch verschiedene biobasierte Noxen, wie Viren, Bakterien, pflanzliche Noxen, Pollen oder Allergene (siehe Tabelle 1) entzündlich verändert sein. Eine allergische Erkrankung an den Konjunktiven ist die allergische Rhinokonjunktivitis.

Insbesondere bei Psoriasis, Dermatitis und atopischer Dermatitis sowie verwandter Hauterkrankungen mit einer Barrierestörung weisen die Epithelgewebe insbesondere in der frühen Phase eine verminderte selektive Permeabilitätsbarriere auf, unter anderem begründend auf einer herunterregulierten Claudin-Expression.

Die vorgenannten Krankheiten können ebenfalls als arbeitsmedizinische Krankheiten infolge einer erhöhten Belastung durch biobasierte Noxen (Tabelle 1) am Arbeitsplatz und/oder infolge einer erhöhten Sensibilität des Menschen auftreten.

In einer weiteren Ausführungsform der erfindungsgemäßen Verwendung des mindestens einen kompatiblen Soluts oder Solutgemisches der Formel I und/oder der Formel II intrudiert die mindestens eine biobasierte, vorzugsweise peptidbasierte, Noxe das äußere Epithelgewebe, insbesondere Epidermis, vorzugsweise das Oberflächenepithel der Haut, das mehrschichtige Epithel der Mundschleimhaut, der Zungenschleimhaut, der Nasenschleimhaut, der Hornhaut oder der Bindehaut des Auges. Durch die Einwirkung der biobasierten, vorzugsweise peptidbasierten Noxen, kann eine Modulation, Veränderung und/oder Schädigung der selektiven Permeabilitätsbarriere, insbesondere der Tight junction, zu einem Eindringen der Noxen zumindest in das Stratum corneum und zumindest teilweise in das Stratum granulosum und/oder in tiefere Epithelschichten erfolgen. Analoge Einwirkungen der Noxen können auf allen Epithelgeweben (Tabelle 2) erfolgen.

Das vorbeschriebene kompatible Solut oder Solutgemisch, vorzugsweise Ectoin und/oder Hydroxyectoin, ist besonders geeignet zur Verwendung bei der Prävention oder Behandlung von durch mindestens eine biobasierte Noxe (Tabelle 1) verursachter Krankheiten von Epithelgeweben (Tabelle 2) umfassend
(1) Infektionen umfassend parasitäre, bakterielle, virale und/oder durch Pilze (Tabelle 1) ausgelöste Infektionen. Solche Infektionen umfassen insbesondere Infektionen der äußeren Epithelgewebe, wie Hautinfektionen umfassend Erythrasma, Impetigo contiaguisa (Flechte), Herpes Labialis, Phlegmone, Furunkel, Hauttuberkulose und Tinea pedis (Fußpilz); Infektionen der Übergangsepithelgewebe, wie obere Atemwege und Mundhöhle, umfassend infektiöse Nasennebenhöhlenentzündung, infektiöse Zahnfleischinfektionen, bakterielle Parodontitis; der inneren Epithelgewebe, wie Atemwegsinfektionen umfassend infektiöse Bronchitis, Bronchiolitis und/oder Alveolitis;
(2) Allergien, welche eine Immunreaktion des Körpers auf nicht-infektiöse Noxen (Antigene bzw. Allergene) (Tabelle 1) mit Entzündungszeichen auslösen, umfassend Allergien der äußeren Epithelgewebe, wie Urtikaria, Kontaktekzeme, Neurodermitis und allergische Konjunktivitis; der Übergangsepithelgewebe, wie Rhinitis allergica, allergische Rhinopathie und Sinusitis; der inneren Epithelgewebe, wie Asthma bronchiale, und
(3) mechanische Läsionen, welche durch Umweltnoxen (Tabelle 1), wie hohe Temperatur, extreme Kälte, geringe Luftfeuchtigkeit und/oder Wind verursacht werden und umfassen trockene, spröde und/oder rissige äußere Epithelgewebe, wie Haut Horn- und/oder Bindehaut des Auges, eingerissene Mundwinkel, rissige Lippen und Sonnenbrand, trockene Übergangsepithelgewebe, wie trockene Schleimhäute der Nase. Solche mechanische primäre Beeinträchtigungen der Epithelgewebe können bereits zur Schädigung der Barrierefunktion der betroffenen Epithelgewebe führen und eine sekundäre Schädigung durch weitere biobasierte, vorzugsweise peptidbasierte, Noxen (Tabelle 1, a), b), c), d) und/oder e)) resultieren.

Primäre Beeinträchtigungen und/oder Schädigungen der Barrierefunktion von Epithelgeweben im Sinne der Erfindung sind solche die zuerst auftreten, phänotypisch nicht zwingend bemerkt werden und nicht pathologisch sind. Zur Prävention oder Behandlung solcher Barrieredefekte ist ggf. ein Kosmetikum oder Medizinprodukt geeignet. Aus solchen primären Barrieredefekten können sekundäre Beeinträchtigungen und/oder Schädigungen der Barrierefunktion von Epithelgeweben entstehen. Sekundäre Barrieredefekte im Sinne der Erfindung sind solche, welche auf einen primären Barrieredefekt folgen und zu einer weiteren Schädigung der Barrierefunktion führen, die phänotypisch deutlich erkennbar und charakterisierbar sind. Dies ist häufig der Fall, wenn durch Umweltnoxen mechanische Läsionen gemäß der oben beschriebenen Gruppe (3) auftreten und anschließend durch infektiöse Noxen (siehe Gruppe (1) oben) Infektionen oder durch nicht-infektiöse Noxen (siehe Gruppe (2) oben) Allergien verursacht werden. Häufig sind sekundäre Barrieredefekte pathologisch und müssen mit einem Medizinprodukt oder Arzneimittel behandelt werden. Im Sinne der Erfindung können sekundäre Barrieredefekte auch ohne primäre Barrieredefekte auftreten. Die jeweils auftretenden Symptome und phänotypischen Erscheinungen wurden bereits oben beschrieben. Für die vorgenannten Gruppen gelten die nachfolgend beschriebenen bevorzugten Ausführungsformen des mindestens einen kompatiblen Soluts, Solutgemisches und/oder Zusammensetzungen sowie Konzentrationen entsprechend.

In einer erfindungsgemäßen Ausführungsform wird das mindestens eine kompatibles Solut oder Solutgemisch ausgewählt aus S-Ectoin, R-Ectoin, (S,S)-Hydroxyectoin, (S,R)-Hydroxyectoin, (R,S)-Hydroxyectoin, (R,R)-Hydroxyectoin und S-Homoectoin, den physiologisch verträglichen Salzen von S-Ectoin, R-Ectoin, (S,S)-Hydroxyectoin, (S,R)-Hydroxyectoin, (R,S)-Hydroxyectoin, (R,R)-Hydroxyectoin und S-Homoectoin, den Amiden und Estern der vorgenannten Verbindungen oder ein Solutgemisch aus mindestens zwei der vorgenannten Verbindungen.

Bevorzugt ist ein kompatibles Solut oder ein Solutgemisch umfassend mindestens zwei der genannten Verbindungen der vorangehenden Definition, wobei die mindestens eine Verbindung ausgewählt wird aus S-Ectoin, R-Ectoin, (S,S)-Hydroxyectoin, (S,R)-Hydroxyectoin, (R,S)-Hydroxyectoin, (R,R)-Hydroxyectoin und S-Homoectoin, den physiologisch verträglichen Salzen von S-Ectoin, R-Ectoin, (S,S)-Hydroxyectoin, (S,R)-Hydroxyectoin, (R,S)-Hydroxyectoin, (R,R)-Hydroxyectoin und S-Homoectoin, den Amiden und Estern der vorgenannten Verbindungen oder ein Solutgemisch aus mindestens zwei der vorgenannten Verbindungen. Ein S-Enantiomer gemäß CIP-Regel entspricht dem L-Enantiomer gemäß der Fischer-Projektion und ein R-Enantiomer gemäß CIP-Regel entspricht dem D- Enantiomer gemäß der Fischer-Projektion. Besonders bevorzugte Verbindungen der Formel I und der Formel II zur Verwendung bei der Prävention oder Behandlung von mit mindestens einer biobasierten Noxe assoziierter Barrieredefekte von Epithelgeweben, insbesondere von Krankheiten umfassend mindestens einen Barrieredefekt in mindestens einer Zelllage mindestens eines Epithelgewebes, sind die vorgenannten Solute.

In einer bevorzugten Ausführungsform des erfindungsgemäßen Soluts oder Solutgemisches zur Verwendung bei der Prävention oder Behandlung von mit biobasierten Noxen assoziierten Barrieredefekten von Epithelgeweben liegt das mindestens eine kompatible Solut in Enantiomer reiner Form mit einer Reinheit von größer gleich 90 % vor, vorzugsweise größer gleich 95 %, größer gleich 97 %, größer gleich 99 %, besondere bevorzugt gleich 100%. Auf das Solutgemisch bezogen, bedeutet das, dass das Gemisch von zwei Verbindungen, die jeweilige Verbindungen in Enantiomer reiner Form aufweist und vorzugsweise keine Verunreinigung der ausgewählten Verbindungen durch das Isomer aufweist. Enantiomer reine Formen des erfindungsgemäßen Soluts oder Solutgemisches weisen besonders bevorzugt S- und/oder (S,S)-Isomere auf.

Bevorzugte Formen des erfindungsgemäßen Soluts sind S(L)-Ectoin, R(D)-Ectoin, (S,S)-Hydroxyectoin, (S,R)-Hydroxyectoin, (R,S)-Hydroxyectoin und (R,R)-Hydroxyectoin sowie Solutgemische aus mindestens zwei der vorgenannten Verbindungen.

In einem bevorzugten Enantiomer reinen Solutgemisch liegen S-Ectoin und (S,S)-Hydroxyectoin jeweils mit einer Reinheit größer gleich 90 % vor, größer gleich 95 %, vorzugsweise größer gleich 97%, größer gleich 99 %, besondere bevorzugt gleich 100 %. Somit weist dieses Solutgemisch bevorzugt jeweils kleiner gleich 10 % kleiner gleich 5 %, vorzugsweise kleiner gleich 3 %, kleiner gleich 1 %, besonders bevorzugt gleich 0 % R-Ectoin oder (R,S)-/(S,R)- oder (R,R)-Hydroxyectoin auf.

In einer besonderen Ausführungsform der Erfindung sind folgende Racemate bevorzugt:
- S-Ectoin und R-Ectoin,
- (S,S)- und (S,R)-Hydroxyectoin, (R,S)- und (R,R)-Hydroxyectoin, oder
- S-Homoectoin und R-Homoectoin.

Das erfindungsgemäße Solutgemisch umfasst mindestens zwei Verbindungen nach Formel I und/oder Formel II und/oder ihre jeweiligen Enantiomere. Das vorbeschriebene erfindungsgemäße Solutgemisch zur Verwendung bei der Prävention oder Behandlung von mit biobasierten, vorzugsweise peptidbasierten, Noxen assoziierter Barrieredefekten von Epithelgeweben umfasst, bezogen auf die Summe aller Verbindungen in dem Solutgemisch mit einem Gesamtgehalt von 100 Gew.-%,
- einen Anteil an S-Ectoin von größer gleich 50 Gew.-% bis kleiner gleich 100 Gew.-% und einen Anteil (S,S)-Hydroxyectoin von größer gleich 50 Gew.-% bis kleiner gleich 100 Gew.-% und bevorzugt
- einen Anteil an S-Ectoin von größer gleich 75 Gew.-%, vorzugsweise größer gleich 85 Gew.-% bis kleiner gleich 95 Gew.-% und einen Anteil (S,S)-Hydroxyectoin von größer gleich 5 Gew.-% bis kleiner gleich 25 Gew.-%, vorzugsweise kleiner gleich 15 Gew.-%.

In einer besonderen Ausführung des erfindungsgemäßen Solutgemisches beträgt
- der Anteil an S-Ectoin kleiner gleich 70 Gew.-%, vorzugsweise kleiner gleich 65 Gew.-%, kleiner gleich 60 Gew.-%, kleiner gleich 55 Gew.-%, besonders bevorzugt größer gleich 50 Gew.-% und
- der Anteil an (S,S)-Hydroxyectoin größer gleich 30 Gew.-%, vorzugsweise größer gleich 35 Gew.-%, größer gleich 40 Gew.-%, größer gleich 45 Gew.-%, besonders bevorzugt kleiner gleich 50 Gew.-%.

In einer Ausführungsform umfasst das Solutgemisch eine Mischung aus zwei Verbindungen der Formel I und/oder der Formel II mit einem Gehalt jeweils größer gleich 60 Gew.-% der ersten Verbindung zu kleiner gleich 40 Gew.-% der zweiten Verbindung. Bevorzugt enthält dieses Solutgemisch größer gleich 60 Gew.-%, besonders bevorzugt größer gleich 70 Gew.-% S-Ectoin und kleiner gleich 40 Gew.-%, besonders bevorzugt kleiner gleich 30 Gew.-% (S,S)-Hydroxyectoin. In einer besonderen Ausführungsform umfasst das Solutgemisch eine Mischung aus zwei Verbindungen der Formel I und/oder der Formel II mit einem Gehalt von 50 Gew.-%, S-Ectoin und 50 Gew.-% (S,S)-Hydroxyectoin.

In einer besonderen Ausführung der erfindungsgemäßen Verwendung bei der Prävention oder Behandlung von mit biobasierten Noxen (Tabelle 1) assoziierten Barrieredefekten von Epithelgeweben (Tabelle 2) ist das mindestens eine Solut der Formel I und/oder der Formel II oder das Solutgemisch enthaltend mindestens zwei Solute der Formel I und/oder der Formel II biobasiert und ist damit biologischen Ursprungs.

Biobasiert oder biologischen Ursprungs im Sinne der Erfindung heißt, dass die Verbindung der Formel I und/oder der Formel II durch bzw. in einem Organismus produziert wird. Bevorzugt ist der Organismus ein Mikroorganismus und besonders bevorzugt ist der Mikroorganismus ein halophiles Bakterium umfassend *Ectothiorhodospira halochloris, Halomonas elongata, Marinococcus halophilus, Brevibacterium linens, Halomonas SPC1, Volcaniella eurihalina, Deleya Salina, Bacillus pantothenticus, Bacillus halophilus, Vibrio costicola* und *Streptomyces parvulust.* Im Sinne der Erfindung wird das biobasierte Solut der Formel I und/oder der Formel II, oder das bereits beschriebene Solutgemisch, vorzugsweise S-/R-Ectoin und/oder (S,S)-/(S,R)-/(R,S)-/(R,R)-Hydroxyectoin, in *Halomonas elongata, Brevibacterium lines oder Marinococcus halophilus* produziert und wird aus diesen Bakterien gewonnen.

Verbindungen der Formel I oder der Formel II, sowie das Solutgemisch umfassend S-Ectoin und (S,S)-/(S,R)-/(R,S)-/(R,R)-Hydroxyectoin, die in biotechnologisch modifizierten Organismen, vorzugsweise in rekombinanten Mikroorganismen umfassend, aber nicht limitiert auf die die vorgenannten Stämme, produziert werden, sind ebenfalls biobasiert oder biologischen Ursprungs, da die vorgenannten Verbindungen durch die Ausstattung der biologischen Zelle hergestellt werden und nicht synthetisch ohne Mitwirkung eines biologischen Organismus im Chemielabor. Verbindungen der Formel I oder der Formel II, mit identischer Struktur, die synthetisch außerhalb eines des vorgeschriebenen Organismus hergestellt werden, gehören nicht der Definition der biobasierten Solute an.

Ein weiterer Gegenstand der vorliegenden Erfindung ist eine pharmazeutische Zusammensetzung enthaltend mindestens ein kompatibles Solut, insbesondere der vorbeschriebenen Art, oder ein Solutgemisch umfassend mindestens zwei kompatible Solute zur Verwendung bei der Prävention oder Behandlung von mit mindestens einer biobasierten, vorzugsweise peptidbasierten, Noxe (Tabelle 1) assoziierter Barrieredefekte von Epithelgeweben, insbesondere Krankheiten umfassend mindestens einen Barrieredefekt, vorzugsweise in mindestens einer Zelllage, in mindestens einem Epithelgewebe. Dabei umfasst das kompatible Solut oder das Solutgemisch mindestens eine Verbindung ausgewählt aus Verbindungen der Formel I, der Formel II, den physiologisch verträglichen Salzen der Formel I und Formel II, den stereoisomeren Formen der Verbindungen der Formel I, Formel II und der physiologisch verträglichen Salze der stereoisomeren Formen oder einem Gemisch aus mindestens zwei der vorgenannten Verbindungen. Die Definition der Formel I und Formel II sowie ihrer Reste R1, R2, R3, R4 und R5 sowie n und Alkyl sind bereits oben definiert. Die Definitionen und bevorzugten Reste, bevorzugten Verbindungen und Kombinationen gelten für die Zusammensetzung entsprechend. Besonders bevorzugt sind S(L)-Ectoin, R(D)-Ectoin, (S,S)-Hydroxyectoin, (S,R)-Hydroxyectoin, (R,S)-Hydroxyectoin und/oder (R,R)-Hydroxyectoin.

In einer weiteren Ausführungsform der erfindungsgemäßen Verwendung der vorgenannten Zusammensetzung liegt das mindestens eine kompatible Solut, vorzugsweise Ectoin und/oder Hydroxyectoin, oder das Solutgemisch mit einem Anteil von größer gleich 0,0001 Gew.-% bis kleiner gleich 50 Gew.-% in der Zusammensetzung vor, bezogen auf den Gesamtgehalt der Zusammensetzung.

Bevorzugte Zusammensetzungen enthalten das mindestens eine kompatible Solut oder das Solutgemisch, vorzugsweise S(L)-Ectoin, R(D)-Ectoin, (S,S)-Hydroxyectoin, (S,R)-Hydroxyectoin, (R,S)-Hydroxyectoin und/oder (R,R)-Hydroxyectoin, mit einem Anteil von größer gleich 0,0001 Gew.-% bis kleiner gleich 50 Gew.-% in der Zusammensetzung, bezogen auf den Gesamtgehalt der Zusammensetzung. Besonders bevorzugt ist ein Bereich größer gleich 0,001 Gew.-%, größer gleich 0,01 Gew.-%, größer gleich 0,1 Gew.-%, besonders bevorzugt größer gleich 1,0 Gew.-% bis kleiner gleich 40 Gew.-%, kleiner gleich 30 Gew.-%, kleiner gleich 20 Gew.-%, besonders bevorzugt kleiner gleich 10 Gew.-%

In einer weiteren Ausführungsform der erfindungsgemäßen Verwendung der vorgenannten Zusammensetzung liegt das mindestens eine kompatible Solut oder das Solutgemisch, vorzugsweise S(L)-Ectoin, R(D)-Ectoin, (S,S)-Hydroxyectoin, (S,R)-Hydroxyectoin, (R,S)-Hydroxyectoin und/oder (R,R)-Hydroxyectoin, mit einem Anteil von größer gleich 0,0001 Gew.-% bis kleiner gleich 10 Gew.-% in der Zusammensetzung vor, bezogen auf den Gesamtgehalt der Zusammensetzung.

In einer besonders bevorzugten erfindungsgemäßen Zusammensetzung liegt das mindestens eine kompatible Solut oder das Solutgemisch mit einem Anteil größer gleich 0,0001 Gew.-% bis kleiner gleich 10 Gew.-% in der Zusammensetzung vor, bezogen auf den Gesamtgehalt der Zusammensetzung, vorzugsweise größer gleich 0,001 Gew.-% bis kleiner gleich 8 Gew.-%, vorzugsweise bis kleiner gleich 6 Gew.-%, besonders bevorzugt kleiner gleich 5 Gew.-%.

Bevorzugt umfasst die erfindungsgemäße Zusammensetzung mindestens eine Verbindung der Formel I und/oder der Formel II ausgewählt aus S-Ectoin, R-Ectoin, (S,S)-Hydroxyectoin, (S,R)-Hydroxyectoin, (R,S)-Hydroxyectoin, (R,R)-Hydroxyectoin und S-Homoectoin, den physiologisch verträglichen Salzen von S-Ectoin, R-Ectoin, (S,S)-Hydroxyectoin, (S,R)-Hydroxyectoin, (R,S)-Hydroxyectoin, (R,R)-Hydroxyectoin und S-Homoectoin, den Amiden und Estern der vorgenannten Verbindungen oder ein Solutgemisch aus mindestens zwei der vorgenannten Verbindungen ist. Besonders bevorzugt liegt mindestens eines der vorgenannten kompatiblen Solute oder das Solutgemisch mit einem Anteil größer gleich 0,0001 Gew.-% bis kleiner gleich 10 Gew.-% in der Zusammensetzung vor, bezogen auf den Gesamtgehalt der Zusammensetzung.

In einer weiteren Ausführungsform der erfindungsgemäßen Verwendung der vorgenannten Zusammensetzung und/oder des erfindungsgemäßen Soluts oder Solutgemisches, insbesondere jeweils zur Verwendung bei der Prävention oder Behandlung von mit mindestens einer biobasierten Noxe assoziierter Barrieredefekte von Epithelgeweben, liegt das Solut, Solutgemisch und/oder die Zusammensetzung als Arzneimittel, Medizinprodukt, Kosmetikum, als Zusatz zu einem der vorgenannten Erzeugnisse und/oder vorzugsweise als Bestandteil von in-vitro-Diagnostikprodukten (IVD) vor. Das Solut oder das Solutgemisch kann zu bestehenden Rezepturen und/oder Formulierungen bestehender Arzneimittel beigemengt werden, um die stabilisierende, schützende (präventive) und/oder fördernde (therapeutische) Wirkung des Soluts auf die Barrierefunktion der Epithelgewebe hinzuzufügen.

In einer weiteren Ausführungsform der erfindungsgemäßen Verwendung der vorgenannten Zusammensetzung und/oder des erfindungsgemäßen Soluts oder Solutgemisches jeweils zur Verwendung bei der Prävention oder Behandlung von mit mindestens einer biobasierten Noxe assoziierter Barrieredefekte von Epithelgeweben, liegt das Solut, Solutgemisch oder die Zusammensetzung in fester oder flüssiger Form oder als Gemisch vor, ausgewählt aus
i) feste Formen umfassend Pulver, Lyophilisat, Tabletten, Granulat, Filmtablette, Dragee, Kapseln, Brausetabletten, Puder und Seife,
ii) flüssige Formen umfassend Lösung, Injektion, Infusion, Tinktur, Tropflösung, Suspension, Emulsion, Gel, Schaum und Creme, und/oder
iii) Gemische umfassend Spray, Aerosole, Salbe, Paste und Kapsel, und insbesondere halbfeste Formen.
Bevorzugt enthalten diese Formen vorzugsweise mindestens ein Solut der Formel I und/oder der Formel II, vorzugsweise S-Ectoin und/oder (S,S)-Hydroxyectoin, besonders bevorzugt mit einem Anteil größer gleich 0,0001 Gew.-% bis kleiner gleich 10 Gew.-% in der Zusammensetzung, bezogen auf den Gesamtgehalt der Zusammensetzung.

Für die orale Verabreichung zur Aufnahme über den Magen-Darm-Trakt durch Schlucken der erfindungsgemäßen Zusammensetzung und vorzugsweise zur Verwendung bei der Prävention oder Behandlungen von Krankheiten der inneren Epithelgewebe von Magen- und/oder Darmschleimhaut umfassend mindestens einen Barrieredefekt, sind besonders bevorzugt
i) feste Formen umfassend Pulver, insbesondere nach Mischen mit einem Getränk oder Wasser, Kapseln, Tabletten, Granulat, Filmtablette, Dragee und Brausetabletten, oder
ii) flüssige Formen umfassend Lösung, Tropflösung, Tinktur, Sirup, Saft und Öl.

Flüssige Formulierungen, vorzugsweise Lösungen, Tinkturen, Spülungen, Lösungen zum Gurgeln und Tropflösungen und/oder Gemische, wie Sprays, werden bevorzugt zur Verwendung bei der Prävention oder Behandlungen von Krankheiten der Übergangsepithelgewebe der Mundhöhle, Nasenhöhle, Rachen und Gaumen jeweils umfassend mindestens einen Barrieredefekt eingesetzt.

Zur Verwendung bei der Prävention oder Behandlungen von Krankheiten der äußeren Epithelgewebe der Haut, Oberhaut, Lippen und Außenohr jeweils umfassend mindestens einen Barrieredefekt, werden bevorzugt feste Formen, wie Pulver, Puder, Seife oder Gemische, wie Salben, Cremes, Gele, Hydrogele eingesetzt.

Jede erfindungsgemäße Zusammensetzung kann in der ausgewählten Formulierung aus dem Stand der Technik bekannte Hilfsstoffe enthalten. Hilfsstoffe umfassen Trägerstoffe, Konservierungsstoffe, Antioxidantien, Stabilisatoren, Lösungsvermittler, Vitamine, Färbemittel, Geruchsverbesserer.

Trägerstoffe, insbesondere für kosmetische Formulierungen und/oder Medizinprodukte, umfassen tierische und pflanzliche Fette, Wachse, Paraffine, Stärke, Traganth, Cellulosederivate, Polyethylenglykole, Silikone, Bentonite, Kieselsäure, Talkum und Zinkoxid oder Gemische aus mindestens zwei der vorgenannten Stoffe. Die vorgenannten Trägerstoffe sind insbesondere geeignet für Salben, Pasten, Cremes und Gele enthaltend mindestens ein Solut der Formel I oder der Formel II, vorzugsweise S-Ectoin und/oder (S,S)-Hydroxyectoin.

Trägerstoffe für Puder oder Sprays enthaltend mindestens ein Solut der Formel I oder der Formel II, vorzugsweise S-Ectoin und/oder (S,S)-Hydroxyectoin umfassen Milchzucker, Talkum, Kieselsäure, Aluminiumhydroxid, Calciumsilikat und Polyamid-Pulver oder Gemische aus mindestens zwei der vorgenannten Stoffe. Sprays können zusätzlich die üblichen Treibmittel, z.B. Chlorfluorkohlenwasserstoffe, Propan/Butan oder Dimethylether, enthalten.

Lösungen und Emulsionen enthaltend mindestens ein Solut der Formel I oder der Formel II, vorzugsweise S-Ectoin und/oder (S,S)-Hydroxyectoin, können die üblichen Trägerstoffe umfassen, wie Lösungsmittel, Lösungsvermittler und Emulgatoren, z.B. Wasser, Ethanol, Isopropanol, Ethylcarbonat, Ethylacetat, Benzylalkohol, Benzytbenzoat, Propylenglykol, 1, 3-Butylglykol, Ole, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische aus mindestens zwei der vorgenannten Stoffe.

Suspensionen enthaltend mindestens ein Solut der Formel I oder der Formel II, vorzugsweise S-Ectoin und/oder (S,S)-Hydroxyectoin, umfassen übliche Trägerstoffe, wie flüssige Verdünnungsmittel, z.B. Wasser, Ethanol oder Propylenglykol, Suspendiermittel, z.B. ethoxylierte Iso-stearylalkohole, Polyoxyethylensorbitester und Polyoxyethylensorbitan- ester, mikrokristalline Cellulose, Aluminiummetahydroxid, Bentonit, Agar Agar und Traganth oder Gemische aus mindestens zwei der vorgenannten Stoffe.

Besonders für die tägliche Anwendung sind Seifen und Lotionen enthaltend mindestens ein Solut der Formel I oder der Formel II, vorzugsweise S-Ectoin und/oder (S,S)-Hydroxyectoin, geeignet, welche Trägerstoffe, wie Alkalisalze von Fettsäuren, Salze von Fettsäurehalbestern, Fettsäureeiweisshydrolysaten, Isothionate, Lanolin, Fettalkohol, Pflanzenöle, Pflanzenextrakte, Glycerin, Zucker oder Gemische aus mindestens zwei der vorgenannten Stoffe enthalten können. Weitere geeignete Hilfsstoffe für die jeweilige Formulierung sind dem Fachmann bekannt.

Zur Prävention oder Behandlung von mit mindestens einer biobasierten Noxe assoziierter Barrieredefekte von Epithelgeweben, insbesondere Krankheiten umfassend mindestens einen Barrieredefekt, werden verwendet
a) kosmetische Formulierungen umfassend Gemische ausgewählt aus Lippenstift, Lippenpflegestift, Puder, Sonnenmilch, Körperlotion und/oder
b) pharmazeutische Zusammensetzungen (Arzneimittel), Medizinprodukte oder IVD, vorzugsweise enthaltend S-Ectoin und/oder (S,S)-Hydroxyectoin, in fester oder flüssiger Form, wobei Formulierung ausgewählt wird aus
   i) feste Formen umfassend Pulver, Tabletten, Granulat, Filmtablette, Dragee, Kapseln, Brausetabletten, Puder und Seife,
   ii) flüssige Formen umfassend Lösung, Injektion, Infusion, Tinktur, Tropflösung, Suspension, Sirup, Saft, Emulsion, Gel, Schaum, Creme, Lotion, Tensid haltiges Reinigungspräparat, Öl und/oder
   iii) Gemische umfassend Spray, Aerosole, Inhalat, Salbe, Paste, Gele und Hydrogele.

Bevorzugte erfindungsgemäße Formulierungen, insbesondere Medizinprodukte, zur Prävention oder Behandlung von mit mindestens einer biobasierten Noxe assoziierter Barrieredefekte von Epithelgeweben, vorzugsweise enthaltend S-Ectoin und/oder (S,S)-Hydroxyectoin, sind zur topischen Verabreichung auf die Hautoberfläche, auf das Auge, auf die Mund- und Nasenschleimhaut geeignete Formulierungen. Diese enthalten besonders bevorzugt S-Ectoin und/oder (S,S)-Hydroxyectoin, mit einem Anteil größer gleich 0,0001 Gew.-% bis kleiner gleich 10 Gew.-% in der Zusammensetzung enthalten, bezogen auf den Gesamtgehalt der Zusammensetzung.

Medizinprodukte im Sinne der Erfindung sind die hierin beschriebenen Formulierungen enthaltend mindestens ein Solut der Formel I oder der Formel II oder das Solutgemisch aus mindestens zwei der vorgenannten Verbindungen, vorzugsweise S-Ectoin und/oder (S,S)-Hydroxyectoin, zur Verwendung bei der Prävention oder Behandlung des Menschen für medizinisch therapeutische Zwecke. Bevorzugt erfüllen die erfindungsgemäßen Medizinprodukte die Definition und Anforderungen der Richtlinie 93/42/EWG und/oder der entsprechenden Regularien in den USA.

In-vitro-Diagnostikprodukte (IVD) im Sinne der Erfindung umfassen mindestens ein Solut der Formel I oder der Formel II oder das Solutgemisch aus mindestens zwei der vorgenannten Verbindungen als Zusatz (synonym Bestandteil) und erfüllen vorzugsweise die Definition und Anforderungen der Richtlinie 98/79/EG und/oder der Regularien in den USA "U.S. gouvernement regulations Title 21: Food and Drugs PART 809 - IN VITRO DIAGNOSTIC PRODUCTS FOR HUMAN USE Subpart A - §809.3 Definitions".

Bevorzugte Hilfsstoffe für Zusammensetzung von Medizinprodukten und pharmazeutische Formulierungen zur Prävention oder Behandlung von Krankheiten umfassend mindestens einen Barrieredefekte in Epithelgeweben in fester oder flüssiger Form umfassen Lactose, Saccharose, Dextrose, Mannitol, Sorbitol, Stärke, Gelatine, Tragant, Pektin, Cellulose, Methylcellulose, Hydroxypropylmethylcellulose (HPMC), Carboxymethylcellulose-Natrium, Ethylcellulose, Hydroypropylmethylcellulosephthalat, Celluloseacetatphthalat, Polyvinylpyrrolidon, Polyvinylalkohol, Polyacrylsäure, Polyethylenglycol, Polyethylenoxid, Natriumdodecylsulfat, Natriumcetylstearylsulfat und Natriumdioctylsulfosuccinat (auch K, Ca-salze).

Bevorzugte Hilfsstoffe für erfindungsgemäße Lösungen und Suspensionen umfassen Dextrose, Mannitol, Tragant, Pektin, Methylcellulose, Hydroxypropylmethylcellulose (HPMC), Carboxymethylcellulose-Natrium, Polyvinylpyrrolidon, Polyvinylalkohol, Polyacrylsäure, Polyethylenglycol, Polyethylenoxid, Natriumdodecylsulfat, Natriumcetylstearylsulfat, Natriumdioctylsulfosuccinat (auch K, Ca-salze) und insbesondere für Suspensionen auch Cellulose.

Halbfeste Formen oder Gemische im Sinne der Erfindung umfassen bevorzugt Hydroxypropylmethylcellulose (HPMC), Carboxymethylcellulose-Natrium, Polyethylenglycol, Polyethylenoxid, Natriumdodecylsulfat, Natriumcetylstearylsulfat und Pektin.

Eine bevorzugte Zusammensetzung eines Medizinproduktes und/oder eines Arzneimittels enthaltend das erfindungsmäße ein Solut der Formel I oder der Formel II oder das Solutgemisch aus mindestens zwei der vorgenannten Solute, vorzugsweise S-Ectoin und/oder (S,S)-Hydroxyectoin, zur Prävention oder Behandlung von mit mindestens einer biobasierten Noxe assoziierter Barrieredefekte in Epithelgeweben umfasst Formulierungen zum Auftragen, Aufbringen, Einmassieren, Aufsprühen und/oder Auflegen auf die betroffene Epitheloberfläche. Solche Erzeugnisse umfassen Nasenspray, Nasentropfen, Nasenbalsam, Augentropfen, Künstliche Tränenflüssigkeit (trockene Augen), Kontaktlinsen, Augenpads (Auflagen), Augengel, Mundspülung, Mundspray, Gele für das Zahnfleisch, Ohrentropfen, Lösung, Spülung, Suspension, Salbe, Creme, Lotion, Paste, Spray, Gelee, Aerosol, Emulsionen (W/O, OW), Hydrogel, Liposomen, mikrosomale Kapseln, Dampfbadkonzentrat. Besonders bevorzugt sind dermatologische Erzeugnisse zur Prävention oder Behandlung von Krankheiten umfassend mindestens einen Barrieredefekt von äußeren Epithelgeweben und/oder Übergangsepithelgeweben. Die vorbeschriebenen bevorzugten Merkmalskombinationen gelten für die genannten Erzeugnisse entsprechend.

### Beschreibung der Figuren (Fig.):

- Fig. 1: Versuchsaufbau zur Bestimmung des transepithelialen Widerstands (TEER); Im Versuchsgefäß (1) mit einem Zellkulturmedium (2) ist eine Einsatz (3) mit einer transparenten ThinCerts-Membran, Porengröße 0,4 µm, angeordnet. Auf der Membran liegt eine einschichtige Zellschicht einer Zellkultur (4) vor, wobei die Membran mit den Zellen vom Zellkulturmedium bedeckt ist. Die einschichtige Zellschicht (4) ist zwischen den beiden Elektroden E1 und E2 angeordnet, wobei eine definierte Stärke (U) eines Gleichstroms (DC) zwischen den Elektroden E1 und E2 angelegt wird, sodass der applizierte Gleichstrom (DC) durch die Zellen fließt.
- Fig. 2: TEER-Werte [Ohm] von Epithelzellen der Mundschleimhautzellen (TR146) nach Inkubation mit 50 mM Ectoin, 0,01% Lecithin oder PBS für 12 Stunden (h).
- Fig. 3: TEER-Werte [Ohm] von Epithelzellen der Mundschleimhautzellen (TR146) nach Inkubation mit 50 mM Ectoin, 0,01% Lecithin oder PBS über Nacht und anschließender Behandlung mit 0,005 % SDS.
- Fig. 4: TEER-Werte [Ohm] von renalen Epithelzellen aus dem Schwein (LLC-PK1) nach Inkubation mit 50 mM Ectoin, 0,01% Lecithin oder PBS über Nacht und anschließender Behandlung mit 0,001 % BAK.
- Fig. 5: TEER-Werte [Ohm] von humanen Keratinozyten (HaCat) nach Inkubation mit 50 mM oder 100 mM Ectoin oder PBS über Nacht und anschließender Trocknung für 5 min bei Raumtemperatur (RT).
- Fig. 6: Versuchsaufbau zum Allergiepräventionsassay (APA): Eine Einsatz (1) mit einer transparente ThinCerts-Membran (3) mit einer Porengröße von 0,4 µm ist in einem Versuchsgefäß (6) mit Zellkulturmedium in einem äußeren Reservoir (5) angeordnet. Auf der Membran (3) liegt eine geschlossene einschichtige epitheliale Zellschicht (2) vor. Der Einsatz trennt ein inneres Reservoir (7) oberhalb der Zellschicht (3) ab. Das Allergen (5) ist z.B. OVA.

Die nachfolgenden Beispiele zeigen den Einfluss von Ectoin auf verschiedene Epithelgewebe und auf die Barrierefunktion der Epithelzellen bzw. Epithelgewebe. Es wird gezeigt, dass Ectoin wirksam bei der Prävention und Behandlung von mit biobasierten Noxen assoziierten Barrieredefekten von Epithelgeweben ist. Ectoin wirkt strukturell auf die Epithelgewebe.

### Beispiele

### Theoretischer Hintergrund zur Barrierefunktion von Epithelgeweben

Eine Diffusionsbarriere bzw. Membranintegrität ist charakterisiert durch Epithelzellen sowie Endothelzellen, welche interzellulare Verbindungen aufweisen. Diese interzellularen Verbindungen trennen die apikale (Lumen) Seite von der basolateralen (Ablumen) Seite der Zellen und bilden durch ihre komplexen Geometrien eine Diffusionsbarriere für eine Vielzahl an Molekülen im parazellulären Durchgang zwischen dem apikalen (luminal) und basolateralen (abluminal) Kompartiment sowie für den Durchgang über den intrazellulären Transportweg.

Diese Diffusionsbarriere wird durch die sogenannten Tight junctions (interzelluläre Verbindungen) gewährleistet, die benachbarte Zellen miteinander verbinden (Abbott NJ, Ronnback L, Hansson E (2006) Astrocyte-endothelial interactions at the blood-brain barrier. Nat Rev Neurosci, 7: 41-53). Tight junction weisen eine komplexe Struktur auf und bestehen aus einer Vielzahl von Komponenten, umfassend integrale Membranproteine (Claudine, Occludine und Junction Adhäsionsmoleküle "JAMs") und periphere Membranproteine. Die Claudin-Proteinfamilie umfasst bisher 24 beschriebene Mitglieder unterschiedlicher Zelltypen. Davon sind 21 als Komponenten von Tight junction in Epithelmembranen von Niere, Leber, Gehirn und intestinales Gewebe bekannt. Claudine werden in homo- sowie hetereotypischen Anordnungen in einzelnen Tight junction gefunden und können in zwei Hauptkategorien unterteilt werden: Poren-abdichtende ("pore-sealing") und Poren-bildende (pore-forming) Claudine. Claudin-1, -3, -4, -5, -7 und -19 sind als Poren-abdichtende ("pore-sealing") Claudine bekannt. Eine erhöhte Expression der Poren-abdichtenden Claudine führt zu einer Zunahme der Dichte von Epithelgeweben, und zu einem erhöhten transepithelialen elektrischen Widerstand (TEER) und zu einer abnehmenden Permeabilität der Epithelgeweben (Khan N, Asif, AR (2015) Transcriptional Regulators of Claudins in Epithelial Tight Junctions. Mediators Inflamm, Volume 2015, Article ID 219843, doi: 10.1155/2015/219843). Auf der Grundlage dieser Systematik wurden die Versuche zum Nachweise der Effekte von Ectoin auf Epithelgewebe durchgeführt.

Eine intakte Diffusionsbarriere bzw. die Bestimmung der Integrität von Epithelzellen oder Endothelzellen kann auf unterschiedliche Weise gemessen werden. Das OECD Guidance Document 28 empfiehlt die Bestimmung z.B. mittels transepithelialen elektrischen Widerstand (TEER), wie in Fig. 1 gezeigt.

Die Zugabe von einigen Verbindungen, z.B. Hydrocortison oder das Phospholipid Lecithin, zu einer Zellkultur bzw. zu Epithelzellen stärken die Membranintegrität, was anhand eines erhöhten elektrischen Widerstands (TEER) in [Ohm] messbar ist. Dagegen gibt es eine Vielzahl an Verbindungen, welche die Tight junction schwächen, wodurch die Membranintegrität abnimmt (Wegener J, Abrams D, Willenbrink W, Galla HJ, Janshoff A (2004) Automated multiwell device to measure transepithelial electrical resistance Under physiological conditions. Biotechniques, 37: 590).

### Material

### Zellkulturmedium:

- MEM-Medium, PAN-Biotech
- DMEM-Medium (high Glucose), PAN-Biotech
- HAMS-F12, PAN-Biotech
- Medium 199, PAN-Biotech

**Tabelle 3: Verwendete Chemikalien und Zellkulturen**

| **Abkürzung** | **Erläuterung** | **Herkunft** |
|---|---|---|
| PBS | Phosphatpuffer | PAN Biotech |
| TR146 | Humane Mundschleimhaut Epithelzellen | Sigma |
| LLC-PK1 | Schweine renale Epithelzellen | DSMZ |
| SIRC | Kaninchen kornealer Epithelzelle | LGC |
| HaCaT | humane Keratinozyten Zelllinie | Uni Münster |
| RPMI-2650 | human Nasenschleimhaut Epithelzellen | CLS |
| RLE | Ratten bronchiale Epithelzellen (Lunge) | IUF Düsseldorf |
| SDS | Sodiumdodecylsulfat, Detergenz | Sigma |
| BAK | Benzalkoniumchlorid, Konservierungsmittel | Sigma |
| OVA | Ovalbumin | Hyglos |
| Lecithin | Positivkontrolle für TEER-Assay | Sigma |
| Claudin-1 ELISA | representatives Protein von Tight junction | Antibodies-Online |
| Ectoin-D | Erfindungsgemäßes Kompatibles Solut gemäß Formel I oder II | bitop AG |
| OVA ELISA | Ovalbumin (nativ) ELISA Kit (Agro-Bio), Repräsentant eines Allergens | Antibodies-Online |

### Beispiel 1: Einfluss kompatibler Solute auf den transepithelialen elektrischen Widerstand (TEER) von Epithelgeweben

### 1.1 Methode

Der Versuchsaufbau ist in Fig. 1 gezeigt. Zur Bestimmung von TEER wird eine definierte Stärke (U) eines Gleichstroms (DC) zwischen zwei Elektroden E1 und E2 angelegt, wobei die einschichtige Zellschicht (Monolayer) zwischen den beiden Elektroden angeordnet ist (Fig. 1), sodass der applizierte Gleichstrom (DC) durch die Zellen fließt. Die Messung von TEER erfolgt mit einem Epithelial Voltometer (EVOM2). Der resultierende Strom I wird gemäß des ohmschen Widerstands R gemessen, wobei R = U/I ist (Benson K, Cramer S, Galla HJ (2013) Impedancebased cell monitoring: Barrier properties and beyond. FluidsBarriers, 10:5.).

Eine Abnahme des transepithelialen elektrischen Widerstands [Ohm] deutet auf eine defekte Barrierefunktion der Epithelzellen und somit auf eine geringere Membranintegrität hin. Auf diese Weise können in einem Vergleich zwischen unbehandelten Epithelzellen (PBS) und behandelten Epithelzellen (SDS, BAK, Lufttrocknung jeweils mit und ohne Ectoin) Aussagen über den schädigenden Einfluss eines Stresses und die schützende Wirkung eines kompatiblen Soluts im Sinne der Erfindung gezeigt werden.

Zu diesem Zweck wurden in *in vitro* Versuchen Epithelzellen auf speziellen Zellkulturträgern mit einer Membran (ThinCerts, Porengröße 0,4 µm) gesät und zu einer geschlossenen, einschichtigen Zellschicht (synonym Epithel-Monolayer) auf der Membran kultiviert (Fig. 1).

Der intakte Epithel-Monolayer wurde jeweils durch eine TEER-Messung kontrolliert. Wurde über zwei Tage kein Anstieg im TEER gemessen, haben die Epithelzellen einen intakten Epithel-Monolayer mit intakter epithelialer Barriere (selektive Permeabilitätsbarriere) gebildet.

Um Effekte von Ectoin auf die Barrierefunktion, insbesondere Membranintegrität, zu messen wurden TR146 Zellen eines intakten Epithel-Monolayers für 12 Stunden (h) im Zellkulturmedium mit PBS (Kontrolle), mit 50 mM Ectoin und mit 0,1 % Lecithin (Positivkontrolle) inkubiert. Direkt nach der Inkubation wurde der TEER gemessen, wie in Fig. 2 gezeigt.

Um anschließend den Effekt von Ectoin auf die Membranstabilität unter Einfluss von Chemikalien zu testen, wurde ein intakter Epithel-Monolayer von Epithelzellen der Mundschleimhaut (TR146) über Nacht in Zellkulturmedium mit PBS (Kontrolle), mit 50 mM Ectoin und mit 0,1 % Lecithin (Positivkontrolle) inkubiert. Anschließend erfolgte eine Stressbehandlung mit 0,005 % SDS und die Veränderung des transepithelialen elektrischen Widerstands [Ohm] wurde stündlich überwacht (Fig. 3).

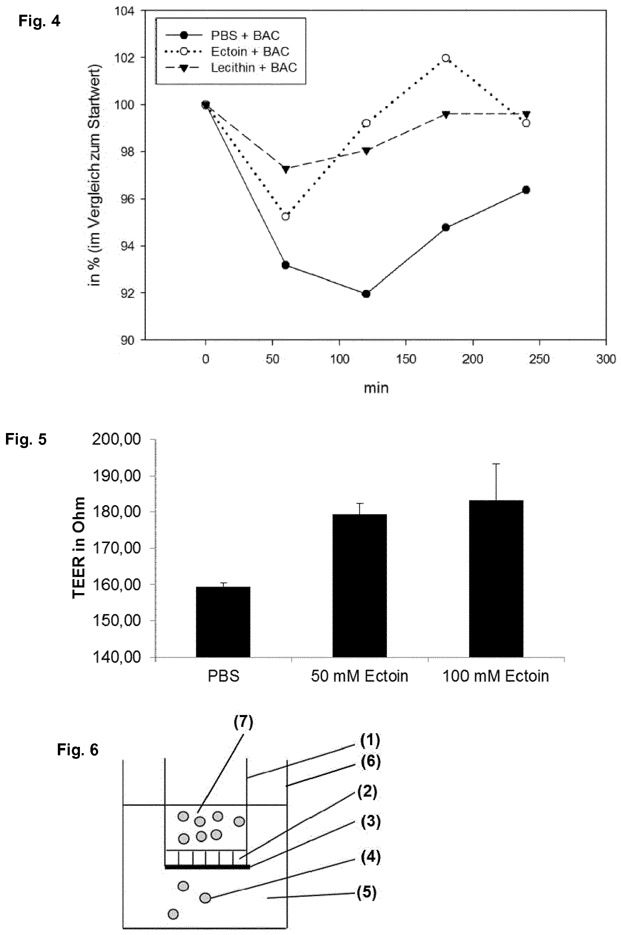

Zusätzlich wurde ein intakter Epithel-Monolayer renaler Epithelzellen aus dem Schwein (LLC-PK1) ebenfalls über Nacht in Zellkulturmedium mit PBS (Kontrolle), mit 50 mM Ectoin und mit 0,1 % Lecithin (Positivkontrolle) inkubiert. Anschließend erfolgte eine Stressbehandlung mit 0,001 % BAK und die Veränderung des transepithelialen elektrischen Widerstands [Ohm] wurde stündlich überwacht (Fig. 4).

In einem weiteren Versuch wurde ein intakter Epithel-Monolayer humaner Keratinozyten (HaCat) ebenfalls über Nacht in Zellkulturmedium mit PBS (Kontrolle), mit 50 mM Ectoin und mit 100 mM Ectoin inkubiert. Anschließend erfolgte eine Stressbehandlung durch Lufttrocknung für 5 min bei Raumtemperatur (RT) unter der Sterilbank (Fig. 5). Nach der Lufttrocknung bei RT unter sterilen Bedingungen, wurden die Zellen wieder mit Medium inklusive OVA-Allergen (250 µg/ml) überschichtet und für 24 h bei 37°C im Brutschrank inkubiert und anschließend wurde der TEER-Wert gemessen.

### 1.2 Ergebnisse

Die Messung des TEERs ist ein direkter Weg, um eine Aussage über die Funktionalität der Barriere von Epithelgeweben, insbesondere zur Membranintegrität und Stabilität, zu erhalten. Eine Senkung des TEERs deutet direkt auf eine gestörte Membran, insbesondere defekte Barriere, hin. Chemikalien, wie SDS und BAK sind in der Lage die Membran zu zerstören, was sich in absinkenden TEER-Werten zeigt. Bei zu hohen Konzentrationen dieser Chemikalien, können die angegriffenen Epithelzellen sich nicht erholen und sterben ab. Die vorbeschriebenen *in-vitro* Versuche weisen einen schützenden und stabilisierenden Effekt von Ectoin auf die Membranstabilität bzw. auf die Barrierefunktion der Epithelzellen anhand von TEER-Werten unterschiedlicher epithelialer Zelllinien nach.

Nach Inkubation mit dem kompatiblen Solut, z.B. Ectoin, waren die TEER Werte [Ohm] jeweils höher als die TEER-Werte der Kontrolle ohne Ectoin (PBS). Zusätzlich zeigen die Versuche einen schützenden Effekt von Ectoin gegen Detergenzien und Konservierungsmittel.

Aus Fig. 2 wird der positive Effekt von Ectoin im Vergleich zu PBS schon in nicht geschädigten Epithelzellen deutlich. Während die epithelialen Mundschleimhautzellen TR146 in PBS einen geringen TEER-Wert von etwa 205 Ohm aufweisen, wird bei den Mundschleimhautzellen nach Inkubation mit 50 mM Ectoin ein mittlerer TEER-Wert von 215 Ohm gemessen. Für 0,1 % Lecithin als Positivkontrolle wird der höchste TEER-Wert mit 225 Ohm gemessen. Somit wurde gezeigt, dass kompatible Solute, insbesondere Ectoin und seine Derivate, die Epithelmembran zusätzlich stabilisieren.

Die Zugabe von Membran zerstörenden Chemikalien wie SDS führte zur starken Abnahme der Membranstabilität, was an der starken Abnahme der TEER-Werte erkennbar ist. Hier führte die Zugabe von 0,005 % SDS zur stärksten Schädigung der epithelialen Mundschleimhautzellen TR146 und zur stärksten Abnahme der TEER-Wert auf 185 Ohm.

Dagegen verhinderte die Inkubation mit Ectoin oder Lecithin (Positivkontrolle) eine Schädigung durch SDS. Die Epithelzellen mit 225 Ohm für Lecithin und 215 Ohm für Ectoin zeigen einen vergleichbaren TEER und auch im Vergleich zu den SDS-geschädigten Zellen (185 Ohm) konnte eine intakte Barriere nachgewiesen werden. Somit wurde durch Ectoin ein stabilisierender Effekt auf die Epithelmembran von Mundschleimhautzellen TR146 erzielt (Fig. 3). Insbesondere wird durch die Präinkubation mit Ectoin ein präventiver und damit schützender Effekt von Ectoin auf die Membran von Epithelzellen der Mundschleimhaut TR146 nachgewiesen.

Entsprechende Ergebnisse wurden in *in-vitro* Versuchen mit Schädigung der Epithelzellen aus dem Schwein (LLC-PK1) durch 0,001 % BAK in Fig. 4 gezeigt. Analog zu Fig. 3 zeigen die Epithelzellen LLC-PK1 bei Schädigung durch BAK (PBS) ohne Ectoin oder Lecithin (Positivkontrolle) die stärkste Abnahme im TEER [Ohm] und damit die stärkste Schädigung der Membranstabilität (Fig. 5). Dagegen wird bereits durch die Zugabe von Ectoin eine Stabilisierung der Membran von Epithelzellen LLC-PK1 erzielt.

In einem weiteren *in-vitro* Versuch wurde der zunächst erhaltenen Epithel-Monolayer von Keratinozyten (HaCat) ohne (PBS) oder mit Ectoin (PBS+Ectoin 50/100 mM) inkubiert und anschließend durch Lufttrocknung gestresst. Danach erfolgte eine Rehydrierung der getrockneten Keratinozyten mit PBS, PBS + Ectoin 50 mM oder PBS + Ectoin 100 mM. Aus Fig. 5 wird deutlich, dass der Trocknungsstress trotz Rehydrierung mit PBS zu einer Schädigung (TEER = ca. 160 Ohm) der Membranstabilität führt. Im Vergleich dazu wird durch eine Präinkubation der Keratinozyten mit Ectoin (TEER = ca. 180 Ohm) eine bessere Membranstabilität erhalten bzw. die Barrierefunktion der Epithelzellen wiederhergestellt. Diese Versuche weisen einen präventiven und einen therapeutischen Effekt von Ectoin auf die Barrierefunktion von Epithelmembranen und damit auf die Membranstabilität nach (Fig. 5).

In allen Versuchen konnte für unterschiedliche epitheliale Zelllinien, wie humane Epithelzellen der Mundschleimhaut TR146, Epithelzellen aus dem Schwein LLC-PK1 und humane Keratinozyten HaCat, jeweils ein stabilisierender Effekt von Ectoin auf den TEER festgestellt werden. Somit ebenfalls ein positiver Effekt von Ectoin auf die Barrierefunktion von Epithelmembranen. Eine Schädigung durch Chemikalien (SDS, BAK) und durch physikalischen Stress (Luft) konnte durch Ectoin verhindert werden. Eine Präinkubation der jeweiligen Epithelzellen mit Ectoin weist somit einen präventiven Effekt von Ectoin nach. Zusätzlich wird durch Zugabe von Ectoin nach einem Stresseinfluss ebenfalls ein die Membranstabilität und die Barrierefunktion wiederherstellender Effekt von Ectoin (Fig. 5) nachgewiesen. Entsprechende Ergebnisse sind bei Stress und Schädigung durch biobasierte Noxen (Tabelle 1) zu erwarten.

Damit ist Ectoin im Sinne der Erfindung sowohl zur Prävention als auch zur Behandlung von Barrieredefekten durch Trockenheit und/oder durch Allergene geeignet (Fig. 2 bis Fig. 5).

### Beispiel 2: Einfluss kompatibler Solute auf die Penetration von Epithelgeweben mit Allergenen - Allergiepräventionsassay (APA)

### 2.1 Methode

Der Versuchsaufbau ist in Fig. 6 gezeigt. Das Zellkulturgefäß (6) umfasst ein inneres (7) und ein äußeres (5) Reservoir, wobei beide Kompartimente durch eine transparente, ThinCert Membran (3) mit einer Porengröße von 0,4 µm getrennt sind. Diese Porengröße lässt Cytokin- und Proteinbewegung zwischen dem äußeren und inneren Reservoir zu (Fig. 6). Eukaryotische Zellen können diese Membran nicht durchdringen und bilden daher eine einschichtige geschlossene Zellschicht, welche die komplette Membran bedeckt. Dadurch wird in diesem Versuchsaufbau eine Anordnung erzielt, welche die apikale Seite (äußeres Reservoir) und basolaterale Seite (inneres Reservoir innerhalb des Einsatzes) der Epithelzellen wiederspiegelt. Allerdings können Allergene, wie z.B. das Ovalbumin (OVA), aufgrund ihrer geringen Molekülgrößere die Membran ohne Probleme passieren.

Liegt eine geschlossene epitheliale Zellschicht vor, kann OVA diese Membranbarriere nur durch "gaps", Tight junction oder Trancytose von der apikalen auf die basolaterale Seite passieren. (Ding L, Zhang Y, Jiang Y, Wang L, Liu B, Liu J (2014) Transport of egg white ACE-Inhibitory peptide, Gln-lle-Gly-Leu-Phe, in human intestinal Caco-2 cell monolayers with cytoprotective effect. J Agric Food Chem (Epub ahead of print)).

### 2.1.1 Methode ohne Trocknungsstress

Die Zellen der jeweiligen Zelllinie (siehe Tabelle 3) wurden auf einer Membran (3) (Fig. 6) bis zur Bildung einer geschlossenen einschichtigen Zellschicht (2) (synonym Epithel-Monolayer) kultiviert. Anschließend wurde der Epithel-Monolayer mit Ectoin (10, 50, 100 mM) für 6 Stunden in Zellkulturmedium vorbehandelt. Nach der Vorbehandlung mit Ectoin wurde der Epithel-Monolayer mit 250 µg/ml des Allergens OVA (Fig. 6), (4) über Nacht inkubiert. Anschließend wurde der OVA-Gehalt im inneren und äußeren Reservoir mittels OVA spezifischem ELISA gemessen und die relative Penetration des Epithel-Monolayers mit OVA in vorbehandelten und nicht behandelten Zellen bestimmt.

### 2.1.2 Methode mit Trocknungsstress

Um trockene Haut zu imitieren, wurde der Epithel-Monolayer auf der Membran für 5 Minuten durch Lufttrocknung und Flüssigkeitsverlust gestresst. Dafür wurden der Zellkulturüberstand entfernt und das Epithel-Monolayer wurde diagonal in einer neuen 12-well Platte fixiert, sodass die Flüssigkeit abfließen konnte. Nach dem Trocknen wurde der Epithel-Monolayer wieder in das Zellkulturmedium überführt und mit Ectoin und Allergen analog zu 2.1.1 behandelt.

### 2.2 Ergebnisse

### 2.2.1 Penetration der Membran mit dem Allergen

Die Penetration des Epithel-Monolayers *in-vitro* mit dem Allergen OVA jeweils mit oder ohne Ectoin-Vorbehandlung wurde mittels eines OVA spezifischen ELISA analysiert. Da jeweils nach Vorbehandlung mit Ectoin im inneren Reservoir (basolaterale Seite) eine deutlich geringere Menge OVA nachgewiesen wurde als im innere Reservoir ohne Vorbehandlung mit Ectoin, führt eine Ectoin Vorbehandlung jeweils zu einer geringeren Penetration des Epithel-Monolayers mit OVA im Vergleich zum Epithel-Monolayer ohne Ectoin-Vorbehandlung. Damit hat wurde für Ectoin in verschiedenen Zelllinien ein die Permeabilitätsbarriere von Epithelgeweben stabilisierender Effekt nachgewiesen.

Insgesamt wurden fünf verschiedene Zelllinien untersucht, welche die unterschiedlichen Körperstellen bzw. Organe repräsentieren, welche mit Allergenen in Kontakt kommen, z.B. Epithelzellen der Mundschleimhaut (TR146), Epithelzellen der Nasenschleimhaut (RPMI-2650), bronchialen Epithelzellen (RLE), Hornhaut des Auges (SIRC) und Oberhaut (HaCat). In allen getesteten Zelllinien konnte ein schützender Effekt für Ectoin auf die Barrierefunktion der Epithelgewebe nachgewiesen werden (Tabelle 4).

Der Tabelle 4 kann entnommen werden, dass im Vergleich zur Penetration mit OVA der jeweiligen Epithel-Monolayer der Mundschleimhaut (TR146) in PBS ohne Ectoin-Vorbehandlung nach einer Vorbehandlung mit 50 mM und 100 mM Ectoin die Permeabilität des Epithel-Monolayers für OVA um 40 % bis 60 % geringer ist. Bei 10 mM Ectoin ist der die Permeabilitätsbarriere schützende Effekt von Ectoin nur in Epithel-Monolayern der Mundschleimhaut (TR146) und der Bronchien (RLE) signifikant im ELISA messbar. In den übrigen epithelialen Zelllinien konnte für eine Vorbehandlung für 6 Stunden mit 10 mM Ectoin im Vergleich zu nicht behandelten Zellen (PBS) in diesem Versuchsaufbau mittels ELISA nur ein sehr geringer oder kein signifikanter stabilisierender Effekt nachgewiesen werden.

Deutliche Effekte sind bei einer Vorbehandlung der Epithel-Monolayer mit 50 mM Ectoin zu sehen. Hier werden, mit Ausnahme der RLE-Zellen, mindestens 20 % bis 50 % des Allergens OVA an der Penetration und an dem Durchdringen der Epithelmembran von der apikalen Seite auf die basolaterale Seite durch die Einwirkung von Ectoin gehindert (Tabelle 4). Bei SIRC-, HaCat- und RPMI-Zellen, welche jeweils mit 100 mM Ectoin vorbehandelt wurden, werden ebenfalls bis zu 50 % des Allergens OVA durch die Einwirkung von Ectoin auf die Membran zurück gehalten und gelangen nicht auf die basolaterale Seite des Epithel-Monolayers.

Somit wurde gezeigt, dass Ectoin in der Lage ist, die Membranstruktur und die Barrierefunktion von Epithelgeweben zu stabilisieren und die Penetration von Allergenen zu inhibieren.

### 2.2.2 Penetration der Membran mit Allergenen nach Trocknungsstress

In einem weiteren *in vitro* Versuch wurde die OVA-Penetration getrockneter Epithelzellen gemäß 2.1.1 mittels ELISA gemessen. Es wurde gezeigt, dass nach Trocknung und Rehydrierung des HaCat-Epithel-Monolayers ohne die Zugabe von Ectoin (PBS) OVA auf die basolaterale Seite (Lumen) penetrieren konnte (Tabelle 4). Dagegen führt die Vorbehandlung mit 50 mM Ectoin bereits zu einer Wiederherstellung der Membranbarriere, sodass durch 50 mM Ectoin eine Inhibierung der Penetration von OVA um 12 % erzielt wird. Im Vergleich dazu wurde beim HaCat-Epithel-Monolayer ohne Trocknungsstress mit 50 mM Ectoin eine Inhibierung der OVA-Penetration um 55 % erzielt (Tabelle 4).

Somit konnte eindeutig in fünf unterschiedlichen Epithel-Zelllinien gezeigt werden, dass Ectoin die Zellmembran stabilisiert. Sowohl in der Haut als auch in der Mundschleimhaut konnte die Penetration der Epithelzellschicht mit Noxen durch Ectoin inhibiert werden. Durch die Stabilisierung der Membran, wird die Barrierefunktion der gesamten Zellschicht gegen die Penetration von Allergenen gestärkt. Somit hat Ectoin einen schützenden Effekt.

**Tabelle 4: Relativer Penetrationsschutz gegenüber dem Allergen OVA**

| **Epithel-Monolayer der Zellkultur** | **Behandlung** | **Penetrationsschutz (im Vergleich zu PBS) [%] +/- Stabw** |
|---|---|---|
| SIRC | OVA + PBS | 0 +/-5,64 |
| | OVA + 10 mM Ectoin | 0 +/-11,52 |
| | OVA + 50 mM Ectoin | 16,3 +/- 2,31 |
| | OVA + 100 mM Ectoin | 29,5 +/- 16,99 |
| HaCaT | OVA + PBS | 0 +/-13,33 |
| | OVA + 10 mM Ectoin | -0,61 +/- 0,1 |
| | OVA + 50 mM Ectoin | 43,5 +/- 13,01 |
| | OVA + 100 mM Ectoin | 52,4 +/10,44 |
| TR146 | OVA + PBS | 0 +/- 7,01 |
| | OVA + 10 mM Ectoin | 12,07 +/ 2,31 |
| | OVA + 50 mM Ectoin | 32,52 +/ 10,01 |
| | OVA + 100 mM Ectoin | 13,44 +/- 7,69 |
| RPMI | OVA + PBS | 0 +/ 11,1 |
| | OVA + 10 mM Ectoin | 23,5 +/- 17,1 |
| | OVA + 50 mM Ectoin | 69,3 +/- 35,4 |
| | OVA + 100 mM Ectoin | 67,1 +/-14,1 |
| RLE | OVA + PBS | 0 +/- 2,45 |
| | OVA + 10 mM Ectoin | 12,7 +/- 10,4 |
| | OVA + 50 mM Ectoin | -6,3 +/-7,91 |
| | OVA + 100 mM Ectoin | -124,5 +/- 3,72 |
| HaCaT (Trockenstress) | OVA + PBS | 0 +/-7,36 |
| | OVA + 50 mM Ectoin | 8,1 +/-8,01 |
| | OVA + 100 mM Ectoin | 11,6 +/- 6,25 |

Die Versuche zeigen eindeutig einen präventiven Effekt für Ectoin (Tabelle 4). Zusätzlich wurde ein therapeutischer Effekt gezeigt, da sogar bei trockener Haut die Barriere wieder teilweise hergestellt werden konnte.

Damit sind Ectoin und seine Derivate als kompatible Solute im Sinne der Erfindung sowohl zur Verwendung bei der Prävention als auch Behandlung von Barrieredefekten in Epithelgeweben geeignet, insbesondere von Krankheiten mit mindestens einem Barrieredefekt in mindestens einer Zelllage mindestens eines Epithelgewebes. Auch wenn vorliegend diese Versuche nicht durchgeführt wurden, ist zu erwarten, dass Versuche mit künstlichen Hautmodellen (z.B. Phenion model, Henkel) oder in Tierexperimenten die Ergebnisse bestätigen werden.

### Beispiel 3: Einfluss von kompatiblen Soluten auf Tight junction - Claudin-1 Expression 3.1 Methode

Die HaCat-Zelllinie wurde in einem Kunststoff-Zellkulturgefäß kultiviert bis eine geschlossene einschichtige Zellschicht (HaCat-Monolayer) gebildet war. Anschließend wurde der Monolayer mit 50 mM oder 100 mM Ectoin für 6 Stunden (h) im Zellkulturmedium vorbehandelt. Nach der Vorbehandlung erfolgte eine Temperaturbehandlung der Zellen bei 44 °C für 30 min. Nach dem Hitzestress wurde der Monolayer erneut bei 37°C im Brutschrank für 24 Stunden inkubiert. Anschließend wurde der HaCat-Monolayer geerntet und nach Lyse mittels Cryoschock (Einfrieren, Auftauen und Abschaben mit Spatel) wurde das Lysat auf Claudin-1 mittels ELISA analysiert.

### 3.2 Ergebnisse

Nach 24 Stunden Inkubation mit 100 mM Ectoin wurde eine signifikante Zunahme der Expression von Claudin-1 in humanen Keratinozyten (HaCat) nachgewiesen. Im Vergleich dazu zeigten die HaCat-Zellen nach einer Inkubation für 24 Stunden in Zellkulturmedium (Medium) und in PBS ohne Ectoin (PBS) eine jeweils um Faktor 3 geringere Expression von Claudin-1.

**Tabelle 5: Claudin-1 Expression in einem HaCat-Epithel-Monolayer**

| **Claudin-1 Expression** | **Medium** | **Ectoin 50 mM** | **Ectoin 100 mM** |
|---|---|---|---|
| | **[Claudin-1] +/- Stabw.** | | |
| | 0,07 ng/ml +/- 0,013 | -0,28 ng/ml +/- 0,002 | 0,22 ng/ml +/- 0,006 |

Somit induziert Ectoin nach einem Hitzestress eine verstärkte Expression von Claudin-1. Es ist zu erwarten, dass die erhöhte Expression von Claudin-1 mit einer Zunahme von TEER korreliert, was die Stärkung der Barrierefunktion der HaCat-Zellen nach einem Trocknungsstress nachweist.

Die vorliegenden Ergebnisse zeigen, dass kompatible Solute, wie das Ectoin und seine Derivate, die Zellmembran stabilisieren und damit die Barrierefunktion der jeweiligen Epithelzellen, insbesondere Epithelgewebe, stärken. Somit zeigen die vorliegenden Ergebnisse, dass Ectoin das Eindringen von biobasierten Noxen (Tabelle 1), wie Toxinen und Allergenen, inhibiert.

## Patentansprüche

1. Kompatibles Solut oder Solutgemisch zur Verwendung bei der Prävention oder Behandlung von mit mindestens einer biobasierten Noxe assoziierter Barrieredefekte von Übergangsepithelgewebe umfassend Genitalien, enthaltend mindestens eine Verbindung ausgewählt aus Verbindungen der Formel I, der Formel II, den physiologisch verträglichen Salzen der Formel I und Formel II, den stereoisomeren Formen der Verbindungen der Formel I, Formel II und der physiologisch verträglichen Salze der stereoisomeren Formen oder einem Gemisch aus mindestens zwei der vorgenannten Verbindungen,
wobei in Formel I 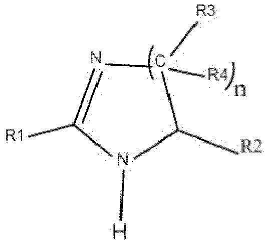 und in Formel II 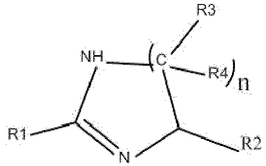
R1 = H oder Alkyl,
R2 = H, COOH, COO-Alkyl oder CO-NH-R5,
R3 und R4 jeweils unabhängig voneinander H oder OH,
R5 = H, Alkyl, ein Aminosäurereste, Dipeptidreste oder Tripeptidreste n = 1, 2 oder 3, und
Alkyl = einen Alkylrest mit C₁-C₄ Kohlenstoffatomen,
bedeuten.

2. Kompatibles Solut oder Solutgemisch zur Verwendung nach Anspruch 1, wobei der mindestens eine Barrieredefekt von mindestens einem Epithelgewebe eine gestörte interzellulare Zellstruktur in mindestens einer Zelllage der Epithelzellen vom Übergangsepithelgewebe aufweist.

3. Kompatibles Solut oder Solutgemisch zur Verwendung nach einem der Ansprüche 1 bis 2, wobei der mindestens eine Barrieredefekt des mindestens einen Epithelgewebes eine gestörte und verminderte selektive Permeabilitätsbarriere ist.

4. Kompatibles Solut oder Solutgemisch zur Verwendung nach einem der Ansprüche 1 bis 3, wobei das das mindestens einen Epithelgewebe ein mehrschichtiges unverhorntes Epithel ist.

5. Kompatibles Solut oder Solutgemisch zur Verwendung nach einem der Ansprüche 1 bis 4, wobei die mindestens eine biobasierte Noxe ausgewählt wird aus
tierischen Noxen, pflanzlichen Noxen, Noxen der Insekten und der Schädlinge, mikrobiellen Noxen, Umweltnoxen, Lebensmittelnoxen, jeweils Bestandteilen und/oder Verbindungen der vorgenannten Noxen und/oder Kombinationen aus mindestens zwei der biobasierten Noxen.

6. Kompatibles Solut oder Solutgemisch zur Verwendung nach einem der Ansprüche 1 bis 5, wobei die mindestens eine biobasierte Noxe oder Kombinationen von mindestens zwei biobasierten Noxen ausgewählt werden aus mindestens einer der Gruppen
a) tierische Noxen umfassend Haustiere, Katze, Hund, Schwein, Nagetiere, Nutztiere, Schwein, Ziege, Absonderungen der vorgenannten Tiere, Tierepithelien und/oder Tierhaare,
b) pflanzliche Noxen umfassend Blütenpollen, Fruchtorgane, Säfte, Sekrete, Gifte, Harze, Naturkautschuk, Latex, Duftstoffe, Aromastoffe, Toxine, Brennhaare, Haken, Nadeln, Dornen, Bestandteile und/oder Verbindungen pflanzlicher Noxen,
c) Noxen der Insekten und der Schädlinge umfassend Milben, Hausstaubmilben, Absonderungen von Insekten, Schädlingen und/oder Parasiten, Bienenharze, Bienengifte, Wespengifte, Spinnengifte, Schädlingsbisse, Mücken-, Bremsen- und Ameisenstiche oder jeweils -bisse, Bestandteile und/oder Verbindungen von Insekten oder Schädlingen,
d) mikrobielle Noxen umfassend Mikroorganismen, Pilze, Hefen, Malassezia-Spezies, Bakterien, Staphyloccocus aureus, Schimmelsporen, Schimmelpilze, bakterielle Toxine, Delta-Toxin, Antibiotika, Viren, Mykotoxine, Bestandteile und/oder Verbindungen der Mikroorganismen,
f) Umweltnoxen wie photodynamische Lichteinwirkung, Wind, saisonale Temperaturschwankungen, und/oder
e) Lebensmittelnoxen umfassend Nüsse, Erdnüsse, Haselnüsse, Wein, Kuhmilch, Weizen, Soja, Hühnerei, Eiweiß, Fisch, Schalentiere, Krebstiere, Weichtiere, rohes Gemüse, rohes Obst, Bestandteile und/oder Verbindungen von Lebensmitteln.

7. Kompatibles Solut oder Solutgemisch zur Verwendung nach einem der Ansprüche 1 bis 6, wobei das mindestens eine Epithelgewebe mit mindestens einem Barrieredefekt, im Vergleich zu einem nicht geschädigten Epithelgewebe, einen reduzierten transepithelialen elektrischen Widerstand (TEER) aufweisen, gemessen in [Ohm].

8. Kompatibles Solut oder Solutgemisch zur Verwendung nach einem der Ansprüche 1 bis 7, wobei das mindestens eine Epithelgewebe mit mindestens einem Barrieredefekt, im Vergleich zu einem nicht geschädigten Epithelgewebe, eine höhere Permeabilität für mindestens eine biobasierte Noxe aufweist.

9. Kompatibles Solut oder Solutgemisch zur Verwendung nach einem der Ansprüche 1 bis 8, wobei das mindestens eine Solut oder Solutgemisch die Barrierefunktion des mindestens einen Epithelgewebes schützt, die Störung der Barrierefunktion zumindest hemmt und/oder zumindest teilweise wiederherstellt.

10. Kompatibles Solut oder Solutgemisch zur Verwendung nach einem der Ansprüche 1 bis 9, wobei der mindestens ein Barrieredefekt des mindestens einen Epithelgewebes phänotypisch umfasst Juckreiz, Hautverfärbungen, Hitze, Hitzeentwicklung, Fieber, Rötungen, trockene Haut, ringförmige Hautveränderungen, Bläschen, Blasen, Pusteln, Papeln, Pickel, jeweils mit und ohne Eiterbildung, Abzesse, Fisteln, Ausschlag, Krusten, Erhebungen, Schwellungen, Kratzer, Stiche, Verschorfung, Schuppung, Quaddeln, Angioodem-Ödem, Quincke-Ödem, Nesselsucht, Plaques, Geschwüre, Furunkel, Karbunkel, Ekzeme und/oder Orientbeulen.

11. Kompatibles Solut oder Solutgemisch zur Verwendung nach einem der Ansprüche 1 bis 10, wobei der mit mindestens einer biobasierten Noxe assoziierte Barrieredefekt mindestens eines Epithelgewebes umfasst
parasitäre, bakterielle und/oder virale Krankheiten, Mykosen, Läsionen, Trockenheit, Reizungen, Entzündungen, Überempfindlichkeiten und/oder allergische Reaktionen der Übergangsepithelgewebe.

12. Kompatibles Solut oder Solutgemisch zur Verwendung nach einem der Ansprüche 1 bis 11, wobei mindestens ein Barrieredefekt vorliegt bei Krankheiten der
- Übergangsepithelgewebe umfassend Entzündungen und Infektionen der Schleimhaut von Genitalien.

13. Kompatibles Solut oder Solutgemisch zur Verwendung nach einem der Ansprüche 1 bis 12, wobei die Krankheiten der Übergangsepithelgewebe umfassen Entzündungen und Infektionen der Genitalien umfassend Haut des Penis, der Eichel, des Skrotum, des Praeputium, Deckschicht der Eichel, Klitorisvorhaut, Klitoriseichel, äußere und inneren Schamlippen

14. Pharmazeutische Zusammensetzung enthaltend mindestens ein kompatibles Solut oder ein Solutgemisch umfassend mindestens zwei kompatible Solute zur Verwendung bei der Prävention oder Behandlung von mit mindestens einer biobasierte Noxe assoziierter Barrieredefekte von Epithelgeweben,
wobei das kompatible Solut oder das Solutgemisch mindestens eine Verbindung umfasst, ausgewählt aus Verbindungen der Formel I, der Formel II, den physiologisch verträglichen Salzen der Formel I und Formel II, den stereoisomeren Formen der Verbindungen der Formel I, Formel II und der physiologisch verträglichen Salze der stereoisomeren Formen oder einem Gemisch aus mindestens zwei der vorgenannten Verbindungen, wobei
in Formel I 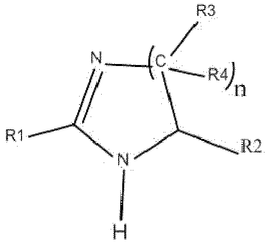 und in Formel II 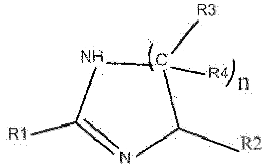
R1 = H oder Alkyl,
R2 = H, COOH, COO-Alkyl oder CO-NH-R5,
R3 und R4 jeweils unabhängig voneinander H oder OH,
R5 = H, Alkyl, ein Aminosäurereste, Dipeptidreste oder Tripeptidreste
n = 1, 2 oder 3, und
Alkyl = einen Alkylrest mit C₁-C₄ Kohlenstoffatomen,
bedeuten.

15. Solut oder Solutgemisch zur Verwendung nach einem der vorgenannten Ansprüchen, und/oder eine Zusammensetzung enthaltend mindestens ein Solut oder ein Solutgemisch nach einem der vorangehenden Ansprüchen, wobei das Solut, Solutgemisch und/oder die Zusammensetzung als Arzneimittel, Medizinprodukt, Kosmetikum und/oder als Zusatz zu einem der vorgenannten Erzeugnisse vorliegt.

16. Solut oder Solutgemisch zur Verwendung nach einem der vorgenannten Ansprüchen, und/oder eine Zusammensetzung enthaltend mindestens ein Solut oder ein Solutgemisch zur Verwendung nach einem der vorangehenden Ansprüchen, wobei das Solut, Solutgemisch und/oder die Zusammensetzung in fester oder flüssiger Form oder als Gemisch vorliegen, ausgewählt aus
i) feste Formen umfassend Pulver, Lyophilisat, Tabletten, Granulat, Filmtablette, Dragee, Kapseln, Brausetabletten, Puder und Seife
ii) flüssige Formen umfassend Lösung, Injektion, Infusion, Tinktur, Tropflösung, Suspension, Emulsion, Gel, Schaum und Creme, und/oder
iii) Gemische umfassend Spray, Aerosole, Salbe, Paste und Kapsel.
